(19) Europäisches Patentamt / European Patent Office / Office européen des brevets

(11) **EP 4 616 875 A1**

(12) **EUROPEAN PATENT APPLICATION**

(43) Date of publication:
**17.09.2025 Bulletin 2025/38**

(21) Application number: **24162814.8**

(22) Date of filing: **12.03.2024**

(51) International Patent Classification (IPC):
***A61L 27/44*** *(2006.01)*

(52) Cooperative Patent Classification (CPC):
(C-Sets available)
**A61L 27/26; A61L 27/44; A61L 27/52;**
A61L 2430/34 (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Novonexile AG**
**4414 Füllinsdorf (CH)**

(72) Inventor: **Tugulu, Stefano**
**4414 Füllinsdorf (CH)**

(74) Representative: **Hepp Wenger Ryffel AG**
**Friedtalweg 5**
**9500 Wil (CH)**

(54) **SEMI-INTERPENETRATING POLYMER NETWORKS**

(57)    A method for producing a semi-interpenetrating polymer network, preferably in the form of a hydrogel, by reacting a first polymer comprising multiple nucleophilic functional groups with a second polymer comprising multiple electrophilic functional groups to produce a covalently cross-linked polymer network in an aqueous environment at a pH value of at least 5.0, preferably at least 6.0, more preferably at least 7.0 in the presence of collagen , preferably native collagen, more preferably native type I collagen, most preferably native homogenised fibrillar type I collagen; and a collagen stabiliser (2) selected from the group consisting of: polysaccharides, preferably selected from the group consisting of alginate, carboxymethylcellulose, chitosan and glycosaminoglycans; gelatin; and poly-L-lactic acid; wherein at least one of the first polymer and the second polymer is at least trifunctional; and the collagen and the collagen stabiliser get non-covalently embedded in the covalently cross-linked polymer network to produce the semi-interpenetrating polymer network.

FIG 4B

(52) Cooperative Patent Classification (CPC): (Cont.)

C-Sets
**A61L 27/26, C08L 5/08;**
**A61L 27/26, C08L 67/04;**
**A61L 27/26, C08L 71/02;**
**A61L 27/26, C08L 89/06**

**Description**

The present invention relates to methods for producing a semi-interpenetrating polymer network, preferably in the form of a hydrogel. It also relates to methods for stabilising collagen in an aqueous environment. It further relates to semi-interpenetrating polymer networks, preferably in the form of hydrogels, as well as kit of parts for their manufacture.

For the regeneration and repair of biological tissue, bioengineered materials are often used, which are inserted in place of the tissue to be regenerated and promote its regeneration. Ideally, the bioengineered material only persists in the body transiently and for a period that is long enough to assist and guide the regeneration of the target tissue before being degraded.

Hydrogels comprising cross-linked polymer networks and a high water content are commonly used for such regenerative biomedical applications as they can be structurally very similar to the native extracellular matrix. Of particular interest are polymer networks that can be produced by an in situ cross-linking (ISC) reaction. This is because they can be injected into the tissue, for example, where the cross-linking reaction takes place to form a corresponding polymer network in the form of a hydrogel, without any major destructive effect on the surrounding tissue. In the cross-linking reaction, the constituents initially present in solution are converted into a form-stable and mechanically robust hydrogel in a solution-to-gel (sol-gel) transition.

Besides physical cross-linking systems, in which the linking is achieved via comparatively weaker ionic bonds or hydrogen bonds, systems with chemical cross-linking are particularly suitable, as these can form a mechanically and chemically more stable polymer network through covalent bond formation.

Chemical cross-linking strategies include enzymatic and radiation-induced cross-linking. However, these types are less suitable for biomedical applications. To be biocompatible, a system should employ bioorthogonal (or at least quasi-bioorthogonal) cross-linking strategies, i.e. it should not be significantly chemically reactive with the biological tissue into which it is introduced; nor should it form significant amounts of by-products that are reactive in such a way. Furthermore, the inserted material should not trigger an immune response in the tissue. In addition, a cross-linking reaction should take place under physiological conditions, in particular at a physiological pH value and temperature of the corresponding tissue, within a clinically acceptable time frame.

Another important characteristic of a suitable polymer network is its degradation properties, which include the type and rate of degradation. In the best case, the degradation rate can be adapted to the specific tissue by changing one or a few parameters and independent of physiological processes and enzymatic activity. This is referred to as a passively degrading polymer network.

The group of polymer networks that fulfils many or even all of these conditions includes poly(ethylene glycol) (PEG) polymers, which are functionalised in such a way that they can be cross-linked with each other as bioorthogonally as possible. For example, Michael-type reactions in which a first polymer comprises $\alpha,\beta$-unsaturated carbonyls, e.g. acrylates, as electrophiles and a second polymer comprises thiol groups as nucleophiles are particularly suitable. The cross-linking reaction between many such electrophiles and nucleophiles takes place at physiological temperatures, in an aqueous environment and around the pH value of 7.0. Certain PEG polymers are known for their tolerable immunogenic profile, meaning they do not tend to cause a strong immune response when integrated in biological tissue. Good and tunable biodegradability, good resorbability, low toxicity and ideal mechanical properties are also advantages of PEG polymers. Also, they can form hydrogels with a high water content (typically 70 % to 90 %). Furthermore, the cross-links originating from the Michael-type reactions involving acrylates are passively degradable hydrolytically and different rates of degradation can be achieved by tuning the concentrations of the polymer precursors.

Such cross-linked PEG polymers, wherein the cross-linking is achieved by a Michael-type reaction between acrylate functional groups and thiol functional groups, are known in the prior art, e.g. in EP 1609491 B1 (STRAUMANN HOLDING AG, 28 December 2005). This document discloses the reaction between a star-shaped PEG-acrylate and a star-shaped PEG-thiol, each having from 2 to 8 arms with functional end groups, to produce a cell-occlusive membrane. The patent holder has also commercialised this technology under the name 'Straumann MembraGel', which is used as a membrane material for dental applications.

Other examples of biomaterials formed by nucleophilic addition reaction to conjugated unsaturated groups are disclosed in EP 1181323 B1 (ETH ZURICH, UNIV ZURICH; 29 June 2011).

Cross-linked PEG polymers as such (like the ones in EP 1609491 B1, STRAUMANN HOLDING AG, 28 December 2005) are not biofunctional. This means that there is a lack of cell adhesion, proliferation and/or cell migration if such polymers were used on their own. However, these properties are essential for a tissue regeneration or repair process. However, cell adhesion is fundamental for the polymer to integrate well in the surrounding tissue and for the regeneration process to take place.

There are numerous ways to improve cell adhesion as well as tissue integration and thus the biofunctionality properties of such a polymer network. One of these is the functionalisation with a cell-binding ligand of at least one of the polymers before the cross-linking reaction. For example, the integrin-binding 'RGD peptide' (RGD = arginine-glycine-aspartic acid) is suitable as a cell-binding ligand, which is known from the prior art (e.g. in TUGULU, S. et al. RGD-functionalized polymer brushes as substrates for the integrin-specific adhesion of human umbilical vein endothelial cells. Biomaterials. 2007, Vol. 28, pages 2536 to 2546; or in DAHLIN, C. et al. Early Biocompatibility of Poly (Ethylene Glycol) Hydrogel Barrier Materials for Guided Bone Regeneration. Clin. Oral Implants Res. 2014, Vol. 25, No. 1, pages 16 to 20).

However, such polymer networks functionalised with cell-binding ligands often exhibit insufficient cell binding despite the ligands. The optimal proportion and the optimal position of the cell-binding ligands in the polymer network are also often decisive for whether the polymer network can be incorporated well in a particular tissue. Adjusting these and other parameters for specific tissues is often very difficult and therefore impractical. In addition, a polymer network that does not have to be individually adjusted and optimised for each biological tissue type would also be much more advantageous.

An alternative strategy to increase the biofunctionality of such polymer networks is the incorporation of biofunctional biomacromolecules into the polymer network. If the biomacromolecules are embedded in the polymer network without covalently binding to it, this is called a semi-interpenetrating network (semi-IPN) .

Collagen is such a biofunctional biomacromolecule, which is also suitable for making a polymer network biofunctional due to its low immunogenicity and commercial accessibility. Collagen and, specifically, type I collagen - the most abundant collagen subtype - comprises 90 % of the protein in human connective tissues. Type I collagen also represents 90 % of the organic bone matrix. It is particularly suitable in tissue regeneration procedures. Collagen type I molecules intrinsically assemble into fibres, which are post-translationally crosslinked, which confers tissue with high mechanical stability. These fibres are ubiquitous throughout the extracellular matrix (ECM) and the various anatomic tissue comprising, e.g. bones, blood vessels, skin, tendons, and fibrous capsules of organs.

The use of collagen alone (without an additional polymer network, for example a PEG polymer network) to produce an ISC hydrogel for regenerative purposes has proven to be disadvantageous. This is because a chemical modification of the collagen is necessary, as the collagen fibres have to be cross-linked. Whilst resulting in mechanically stable hydrogels, this chemical alteration has commonly been associated with inducing undesirable immunogenic properties, rendering direct crosslinking strategies of collagen biologically not or poorly viable for regenerative biomedical applications. Another disadvantage is that the rate of desired degradation of pure collagen matrices (controlled by proteolytic enzymatic activity) is highly dependent on the application, tissue, wound healing phase, inflammatory state, and other factors. It is therefore difficult to control the degradation process, although it would be desirable to be able to adjust the degradation rate in particular depending on the application (FAN, L. et al. The Use of Collagen-Based Materials in Bone Tissue Engineering. Int. J. Mol. Sci. 2013, Vol. 24, page 3744).

It is therefore desirable to embed the collagen chemically unchanged (i.e. in its native state) and thus non-covalently in a polymer matrix to produce a semi-IPN, as described above, with better controllable and reproducible properties. This preserves the low-immunogenic and biofunctional properties of the collagen. Furthermore, the degradation of a polymer/collagen semi-IPN (e.g. a PEG/collagen semi-IPN) would be primarily driven by the degradation kinetics of the polymer network. Provided the degradation mechanism of the latter network would be based on passive and not active enzymatic degradation mechanisms, the resulting hydrogel exhibits physiologically independent, and thus steerable, degradation kinetics.

There are several types of collagen, in particular type I collagen, which differ in terms of the tissue from which they are obtained and how they are processed. Distinct types of soluble collagen (e.g. acid-soluble collagen, pepsin-soluble collagen and Atelo-collagen) are obtained from fibrillar collagen raw materials by acid extraction or enzymatic digestion. However, these soluble collagen types have highly variable structural properties, which are caused by the different processing methods. This leads to very different mechanical and biological properties. This is why these soluble collagen variants are rather undesirable for tissue regeneration applications.

Homogenised fibrillar collagen suspensions are therefore particularly advantageous, as they are essentially chemically unchanged and therefore have the properties of native collagen.

One of the major technical challenges related to the realisation of biofunctional semi-IPN hydrogels with collagen is the problem that collagen (regardless of whether it is dissolved or homogenised fibrillar collagen) is only soluble or stays in a homogenised suspension at pH values below 5.0 (or for certain collagen types below 6.0 or below 7.0). At and above these pH values, the collagen becomes insoluble and/or fibres in suspension form undesirable aggregates, which negatively affects the mechanical and biological properties of the resulting semi-IPN (MEYER, M. Processing of collagen based biomaterials and the resulting materials properties. BioMed Eng OnLine. 2019, Vol. 18, No. 24, pages 1 to 74).

However, these are precisely the pH value ranges that are usually required to carry out the in situ cross-linking (ISC) reactions in reasonable periods of time (seconds to single-digit minutes). However, the collagen must be added to at least one of the two polymer constituents before the ISC reaction so that it is evenly embedded in the resulting semi-IPN. The collagen would form aggregates even before the cross-linking reaction, which would prevent uniform embedding.

For example, the desired pH value for Michael-type reactions between thiols and acrylates is above these values.

Irrespective of the optimum pH range of the cross-linking reaction, it is desirable for biocompatibility reasons that the pH value is not below the specified values when using such a hydrogel in biological tissue regeneration.

It is therefore an object of a first facet of the present invention to overcome at least some, if not all, of the shortcomings of the prior art. In particular, it is an object of the present invention to provide an improved method for producing a semi-interpenetrating polymer network comprising collagen.

The problem is solved by a method for producing a semi-interpenetrating polymer network, preferably in the form of a hydrogel. The problem is further solved by a method for stabilising collagen in an aqueous environment at a pH value of at least 5.0, preferably at least 6.0, more preferably at least 7.0. The problem is also solved by a semi-interpenetrating polymer network obtainable by a method according to the invention; and by a kit of parts for the manufacture of such a semi-interpenetrating network.

Towards this end, a first aspect of the first facet of the invention pertains to a method for producing a semi-interpenetrating polymer network, preferably in the form of a hydrogel. This is done by reacting a first polymer comprising multiple nucleophilic functional groups with a second polymer comprising multiple electrophilic functional groups to produce a covalently cross-linked polymer network in an aqueous environment at a pH value of at least 5.0, preferably at least 6.0, more preferably at least 7.0.

The reaction is performed in the presence of collagen. Preferably, the collagen is native collagen, more preferably native type I collagen, most preferably native homogenised fibrillar type I collagen.

The reaction also takes place in the presence of a collagen stabiliser, which is selected from the group consisting of:

- polysaccharides, preferably selected from the group consisting of alginate, carboxymethylcellulose, chitosan and glycosaminoglycans;
- gelatin; and
- poly-L-lactic acid.

At least one of the first polymer and the second polymer is at least trifunctional, and the collagen and the collagen stabiliser get non-covalently embedded in the covalently cross-linked polymer network to produce the semi-interpenetrating polymer network.

According to the IUPAC definition, a semi-interpenetrating polymer network (semi-IPN) is a polymer comprising one or more polymer networks and one or more linear or branched polymers characterised by the penetration of at least one of the networks by at least some of the linear or branched macromolecules. A semi-IPN is distinguished from an interpenetrating polymer network (IPN) because the constituent linear or branched macromolecules (in the present case, the collagen and the collagen stabiliser) can, in principle, be separated from the constituent polymer network(s) without breaking chemical bonds; it is a polymer blend ('semi-interpenetrating polymer network' in IUPAC Compendium of Chemical Terminology, 3rd ed. International Union of Pure and Applied Chemistry; 2006. Online version 3.0.1, 2019. https://doi.org/10.1351/goldbook.S05598) .

The fact that the collagen and the collagen stabiliser get non-covalently embedded in the covalently cross-linked polymer network means that no covalent bond exists or is formed between each of these compounds and the polymer network. There is also no covalent bond between the collagen and the collagen stabiliser. The collagen and the collagen stabiliser are not chemically modified during the cross-linking reaction between the first polymer and the second polymer.

According to the present invention, a hydrogel is a mixture of porous, permeable solids and at least 20 %-w/w, preferably at least 40 %-w/w, more preferably at least 60 %-w/w, most preferably at least 80 %-w/w of interstitial fluid comprising mainly water, with respect to the total weight of the hydrogel.

In the context of the present invention, the term %-w/w refers to the percentage by weight of a part of a mixture compared to the total weight of the mixture.

Preferably, the first polymer is a branched polymer, preferably a star-shaped polymer, and/or the second polymer is a branched polymer, preferably a star-shaped polymer. A star-shaped polymer is a polymer composed of star macromolecules; a star macromolecule contains a single branch point from which linear chains (arms) emanate. Preferably the multiple nucleophilic functional groups in the first polymer and/or the multiple electrophilic functional groups in the second polymer are located at end points of branches of the branched polymer.

Preferably, the polymer chains of the first and/or the second polymer is/are selected from the group consisting of: cellulose derivatives, polyvinyl pyrrolidone, polyvinyl alcohol, polyacrylic acid, poly (methyl vinyl ether), poly (maleic anhydride), poly (meth)acrylic acid, polyethylene glycols (PEG), polyamides, polyacrylic amides, or copolymers or blends thereof, more preferably the polymer chains of the first and/or the second polymer is/are polyethylene glycols (PEG).

In the context of the present invention, the term "or" is to be understood as an exclusionary disjunction. "A or B" states that exactly one of the two statements is true (if the disjunction is true).

In the context of the present invention, the multiple nucleophilic functional groups of the first polymer are also referred to as such if they only possess the nucleophilicity required for the cross-linking reaction after activation, for example by deprotonation. Likewise, the multiple electrophilic functional groups of the second polymer are also referred to as such if they only possess the electrophilicity required for the cross-linking reaction after activation.

In the context of the present invention, 'reacting' means that the first and second polymers are mixed in an aqueous environment and react with each other, provided that the reaction conditions necessary for the reaction, such as pH and temperature, are present.

In principle, the method according to the invention can be carried out with all possible cross-linking polymers in which the cross-linking reaction takes place at a pH value of at least 5.0, preferably at least 6.0, most preferably at least 7.0; and the first and second polymer must not react with the collagen and/or the collagen stabiliser. It has been shown that the type of polymers play a subordinate role in the invention and can thus be selected to suit the respective application. For tissue regenerative purposes, for example, PEG polymers with thiol and acrylate end groups are suitable for Michael-type bond formation. As described above, the degradation rate of such bonds can be adapted particularly well to the application.

In the context of the present invention, at least trifunctional means that the first polymer and/or the second polymer have at least three nucleophilic functional groups or, respectively, at least three electrophilic functional groups. Preferably, a trifunctional polymer has three branches at each end of which a corresponding functional group is attached. The fact that at least one of the two compounds is trifunctional enables cross-linking for the synthesis of a cross-linked polymer network.

The problem described above of the loss of stability of collagen the more alkaline its aqueous environment, applies to all types of collagen. The solution described below can therefore also be applied to all types of collagen. In particular, the problem applies to native collagen, preferably to native type I collagen, more preferably to native homogenised fibrillar type I collagen.

The term 'native' in the context of native collagen refers to collagen that has been extracted and purified from its original biological source while retaining its natural unaltered structural and biochemical properties. Specifically,

- in native collagen, at least 70 %, preferably at least 80 %, more preferably at least 90 % of the at least one triple-helical domain made of amino acid $\alpha$ chains in natural collagen are still present;
- native collagen does not contain any covalently bound chemical functional groups, which do not occur in natural collagen; and
- in native collagen, there is no covalent cross-linking between the individual collagen fibres that does not occur in natural collagen.

Prior to extraction and purification, the collagen exists as natural collagen in its original biological source.

As described above, the use of native collagen in the context of the present invention is advantageous, as this preserves the biofunctional and low-immunogenic properties of the collagen, in contrast to the use of denatured collagen. These properties are particularly advantageous for applications in biological tissue, for example in tissue regeneration.

Type I collagen is known in the art and is made up of the combination of a proalpha1 and a proalpha2 chain created by the COL1alpha1 (gene symbol for peptide chain: COL1A1) and COL1alpha2 (gene symbol for peptide chain: COL1A2) genes, respectively. As type I collagen is the most abundant collagen in the body, its use is particularly interesting for tissue regeneration applications.

Homogenised fibrillar type I collagen is a homogeneously distributed collagen suspension made from fibrillar collagen raw materials.

Homogenised fibrillar type I collagen is obtainable by a method described in the examples below.

Homogenised fibrillar collagen suspensions display ideal rheological properties with good shear thinning properties. These suspensions contain highly dispersed collagen fibrils considered ideal for even functionalisation of polymer matrices.

Preferably, a fibrillar collagen suspension is used over soluble collagen, as the inventors have found that the use of fibrillar collagen suspension leads to much more reproducible results.

In the context of the present invention, a collagen stabiliser is a compound that prevents collagen from forming aggregates and thus clumping when the collagen is exposed to an aqueous environment above a certain pH value. The critical pH value, above which the collagen becomes unstable, (i.e. the pH value threshold) depends on the type of collagen used, but is preferably 5.0, even more preferably 6.0, most preferably 7.0.

A collagen stabiliser is a polymeric macromolecule.

More than one type of collagen stabiliser can be used. For example, a glycosaminoglycan (e.g. hyaluronic acid) and poly-L-lactic acid can be used together as collagen stabilisers.

Polysaccharides are carbohydrates formed by the linkage of multiple monosaccharides through glycosidic bonds. Alginate is a naturally occurring polysaccharide found in the cell walls of brown algae and it is composed of mannuronic acid and guluronic acid. Carboxymethylcellulose is a derivative of cellulose, where some of the hydroxyl groups of the glucopyranose monomers are etherified with carboxymethyl groups. Glycosaminoglycans are a group of linear polysaccharides that are found in the extracellular matrix of animal tissues. They consist of repeating disaccharide units that typically contain an amino sugar (such as glucosamine or galactosamine) and a uronic sugar (like glucuronic acid or iduronic acid) or galactose. Hyaluronic acid and its derivatives are particularly suitable glycosaminoglycans as collagen stabilisers (see below).

Gelatin is a protein-based polymer derived from collagen, which is found in animal connective tissues.

Poly-L-lactic acid is a synthetic aliphatic polyester, which is biodegradable and biocompatible. It is derived from the naturally occurring lactic acid.

It has surprisingly been found that the collagen stabilisers according to the invention have such an effect and prevent the collagen from forming aggregates and clumping together at a pH value of at least 5.0, preferably at least 6.0, most preferably at least 7.0. This enables the collagen to be evenly embedded in the cross-linked polymer network. If collagen is mixed with a collagen stabiliser and the mixture is then brought to a pH value above the threshold, the undesirable effects that occur in the absence of the collagen stabiliser do not occur.

Without wishing to be bound by any theory, it is believed that individual collagen fibres are stabilised by being embedded and surrounded by the polymer chains from the collagen stabiliser, reducing the interaction between individual collagen fibres. The stabilising interaction between the collagen and the collagen stabiliser is maintained from a pH value of 5.0, preferably 6.0, most preferably 7.0. This prevents the collagen fibres from aggregating and clumping at neutral and basic pH values.

Such a stabilising effect on the collagen was found for all collagen stabilisers mentioned. In addition, the compounds mentioned are generally very biocompatible and have their own favourable biological properties known to the skilled person. This means that the collagen stabiliser can be selected, which not only stabilises the collagen at higher pH values, but also exhibits its own positive properties for the desired application as part of the semi-interpenetrating polymer network.

The exact mechanism of action and the interactions between the collagen and the collagen stabilisers are not yet fully understood. However, without being tied to any one theory, it is assumed that several factors play a role. One such factor is assumed to be that a negatively charged collagen stabiliser in the neutral or alkaline pH range intercalates into the collagen and thus forms a complex, which stabilises the collagen at higher pH values. Another factor that may play a role is the high viscosity of the collagen stabiliser. The molecules of the collagen stabiliser thus have a low mobility, which means that the collagen is also fixed in the matrix of collagen stabiliser molecules.

The use of a collagen stabiliser according to the invention thus enables the non-covalent integration of biofunctional collagen without clumping into a polymer network, which is cross-linked at a pH value that is not actually suitable for the collagen. The advantages of the cross-linked polymer network and the chemically unmodified collagen described above can thus be utilised together, for example for applications in tissue regeneration. The property of the polymer matrix that the degradation rate of the polymer network can be adjusted depending on the application also remains essentially unaffected (given that it is based on a passive, physiologically independent degradation mechanism).

The possibility that chemically unmodified collagen can be embedded in a polymer matrix that is not biofunctional as such, thus making the composition biofunctional (e.g. ensuring good cell adhesion and a low immunogenic profile), is a major advantage over other polymer networks that can be bioorthogonally cross-linked in situ. For example, the hydrogel described in EP 1609491 B1 (STRAUMANN HOLDING AG, 28 December 2005) comprises only an in situ cross-linkable polymer network with comparatively low biofunctionality. This is because it does not contain a biofunctional biomacromolecule such as collagen.

Thanks to the collagen stabiliser, the collagen is homogeneously distributed in the polymer network and this homogeneous distribution gives the resulting hydrogel a particularly high mechanical resistance. This means that it can also be used in tissue regeneration processes and wound healing processes and does not break down if, for example, the tissue is subjected to high mechanical stress during medical wound care.

The problem of providing an improved method for producing a semi-interpenetrating polymer network is thus solved by the method of the first aspect of the invention.

In a preferred embodiment of the first aspect of the invention, before the first polymer is reacted with the second polymer, a stabilised mixture with a threshold pH value of at least 5.0, preferably at least 6.0, more preferably at least 7.0 is prepared. This is either done by combining the collagen with the collagen stabiliser at a pH value below the threshold pH value, before adding a base, preferably sodium hydroxide; and optionally, an aqueous buffer solution, preferably an aqueous phosphate buffer solution. Alternatively, the stabilised mixture is prepared by combining the collagen stabiliser (2) with a base, preferably sodium hydroxide, and optionally, an aqueous buffer solution, preferably an aqueous phosphate buffer solution, before adding the collagen (1). Optionally, at least a part of the stabilised mixture is combined with the first polymer and/or at least a part of the stabilised mixture is combined with the second polymer.

In the context of the present invention, a stabilised mixture is a mixture in which collagen is influenced by a collagen stabiliser in such a way that the collagen does not form aggregates and/or lumps with at least one spatial dimension of at least 30 $\mu$m, preferably at least 70 pm, more preferably at least 100 $\mu$m. Also, there is no phase separation of the mixture optically visible by the naked eye when the pH of its aqueous environment changes from below the threshold pH value to or above this value. A phase separation of the mixture occurs when water accumulates on top of the mixture. Centrifugation of a sample of the stabilised mixture at approximately 3000 rpm (centrifugal force = approximately 500 times the force of gravity) at 1 min does not lead to phase separation of the mixture optically visible by the naked eye.

Combining the collagen with the collagen stabiliser before the pH value is adjusted above the pH value threshold using the base or combining the collagen with a mixture of the collagen stabiliser and the base are both suitable methods for ensuring that a stabilised mixture is produced that can be used at pH values above the pH value threshold.

Preferably, the mass fraction of the added first polymer relative to the total weight of the semi-interpenetrating polymer network is from 1 %-w/w to 20 %-w/w, preferably from 2 %-w/w to 15 %-w/w, more preferably from 4 %-w/w to 10 %-w/w, even more preferably from 5 %-w/w to 7 %-w/w, most preferably is 6 %-w/w.

Preferably, the mass fraction of the added second polymer relative to the total weight of the semi-interpenetrating polymer network is from 1 %-w/w to 20 %-w/w, preferably from 2 %-w/w to 15 %-w/w, more preferably from 4 %-w/w to 10 %-w/w, even more preferably from 5 %-w/w to 7 %-w/w, most preferably is 6 %-w/w.

Preferably the mass ratio between the first polymer and the second polymer is between 0.8:0.2 and 0.2:0.8, preferably between 0.6:0.4 and 0.4:0.6, more preferably is approximately 0.5:0.5.

Preferably, the mass fraction of the added collagen relative to the total weight of the semi-interpenetrating polymer network is from 0.05 %-w/w to 2.00 %-w/w, preferably from 0.10 %-w/w to 1.70 %-w/w, more preferably from 0.20 %-w/w to 1.30 %-w/w, even more preferably from 0.30 %-w/w to 0.90 %-w/w, most preferably from 0.40 %-w/w to 0.50 %-w/w. In a preferred embodiment, the mass fraction of the added collagen relative to the total weight of the semi-interpenetrating polymer network is 0.44 %-w/w.

Preferably, the mass fraction of the added collagen stabiliser relative to the total weight of the semi-interpenetrating polymer network is from 0.10 %-w/w to 10.00 %-w/w, preferably from 0.30 %-w/w to 8.00 %-w/w, preferably from 0.80 %-w/w to 6.00 %-w/w, more preferably from 1.30 %-w/w to 4.00 %-w/w, most preferably from 1.70 %-w/w to 1.80 %-w/w. In a preferred embodiment, the mass fraction of the added collagen stabiliser relative to the total weight of the semi-interpenetrating polymer network is 1.76 %-w/w.

The amount of base is preferably selected so that the desired pH value of the resulting composition is achieved.

Preferably, the aqueous phosphate buffer solution has a pH value ranging from 6.0 to 9.0, preferably ranging from 7.0 to 8.0, more preferably ranging from 7.3 to 7.7. Preferably, the aqueous phosphate buffer solution has a phosphate concentration ranging from 10 mM to 40 mM, preferably ranging from 20 mM to 30 mM, more preferably of 25 mM with respect to the total volume of the stabilised mixture; or the aqueous phosphate buffer solution has a phosphate concentration ranging from 150 mM to 290 mM, preferably ranging from 180 mM to 220 mM, more preferably of 200 mM with respect to the total volume of the stabilised mixture.

In a preferred embodiment of the first aspect of the invention, the first polymer is reacted with the second polymer at a temperature ranging from 22 °C to 47 °C, preferably at a temperature ranging from 27 °C to 42 °C, more preferably at a temperature ranging from 32 °C to 37 °C.

The temperature is preferably selected so that it is within an acceptable temperature range for the application, but of course also so that the cross-linking reaction takes place within a few minutes. In tissue regeneration applications, the reaction temperature is typically determined by the temperature of the surrounding tissue.

The polymerisation time can be determined using rheometric isothermic polymerisation kinetics as described below. The crosslink reaction is considered sufficiently advanced to result in a form-stable elastic cross-linked polymer network, as soon as the storage modulus (G') of the polymer network corresponds to the loss modulus (G'') (see examples below for determining these values).

In a preferred embodiment of the first aspect of the invention, the first polymer comprising multiple nucleophilic functional groups

-    (option I.I) is a polymer comprising multiple thiol functional groups, preferably is a multifunctional polyethylene glycol thiol;
-    (option I.II) is at least tetrafunctional, preferably is tetrafunctional;
-    (option I.III) has a molecular weight in the range of 500 g/mol to 20000 g/mol, preferably in the range of 1000 g/mol to 10000 g/mol, more preferably in the range of 1500 g/mol to 5000 g/mol, even more preferably in the range of 1700 g/mol to 2300 g/mol most preferably in the range of 1900 g/mol to 2100 g/mol; or
-    fulfils a combination of these conditions.

The following combinations of the above options are preferred:

-    option I.I and option I.II;
-    option I.I and option I.III;
-    option I.II and option I.III; or
-    option I.I and option I.II and option I.III.

As described above, thiols are particularly suitable as bioorthogonal reaction partners for the formation of a cross-linked polymer network, especially with an acrylate group as reaction partner of the second polymers. This allows a stable cross-linkage to be generated by a Michael-type reaction. This reaction preferably takes place at a pH value of at least 5.0, preferably at least 6.0, most preferably at least 7.0. Lower pH values lead to a very long reaction time and can cause the mixture to run too strongly before the cross-linking reaction.

Preferably, the cross-linking reaction takes place at a pH value of at most 10.0, preferably at most 9.0, even more preferably at most 8.0 (irrespective of the nucleophilic functional group used). The inventors have found that excessively high pH values lead to too rapid curing (cross-linking), which can result in inhomogeneous mixing and application not being able to take place before the cross-linking reaction has progressed too far.

A multifunctional polyethylene glycol thiol according to the invention has multiple thiol functional groups.

In the context of the present invention, at least tetrafunctional means that the first polymer and/or the second polymer have at least four nucleophilic functional groups or, respectively, at least four electrophilic functional groups. Preferably, a tetrafunctional polymer has four branches at each end of which a corresponding functional group is attached.

A large number of different polymers are suitable as first and second polymers for the present invention. In particular, the invention works with a wide range of molecular weights of the polymers. The higher the molecular weight of the selected polymers, the shorter it takes to degrade the resulting polymer network. The molecular weight of the first polymer (and also of the second, see below) should therefore preferably be selected so that the resulting cross-linked polymer network has a degradation rate suitable for the application. For tissue regeneration, the degradation rate should correspond as closely as possible to the build-up rate of the tissue to be regenerated.

Among other factors, the number of functional groups per molecule and the chain length and arrangement can also influence the degradation rate of the cross-linked polymer network.

In a preferred embodiment of the first aspect of the invention, the second polymer comprising multiple electrophilic functional groups

- (option II.I) is a polymer comprising multiple $\alpha,\beta$-unsaturated carbonyl functional groups, preferably is a polymer comprising multiple acrylate functional groups, more preferably is a multifunctional polyethylene glycol acrylate;
- (option II.II) is at least tetrafunctional, preferably is tetrafunctional;
- (option II.III) has a molecular weight in the range of 500 g/mol to 20000 g/mol, preferably in the range of 1000 g/mol to 10000 g/mol, more preferably in the range of 1500 g/mol to 5000 g/mol, even more preferably in the range of 2000 g/mol to 2600 g/mol, most preferably in the range of 2200 g/mol to 2400 g/mol; or
- fulfils a combination of these conditions.

The following combinations of the above options are preferred:

- option II.I and option II.II;
- option II.I and option II.III;
- option II.II and option II.III; or
- option II.I and option II.II and option II.III.

A multifunctional polyethylene glycol acrylate according to the invention has multiple acrylate functional groups.

In a preferred embodiment of the first aspect of the invention, the reaction between the first polymer and the second polymer is carried out in the presence of at least one further constituent selected from the group consisting of:

- an antimicrobial agent, preferably methyl-4-hydroxybenzoate and/or 1,1'-hexamethylenbis[5-(4-chlorphenyl)biguanid];
- an aqueous buffer solution, preferably an aqueous phosphate buffer solution;
- a base, preferably sodium hydroxide, to neutralize the collagen to form non-charged collagen; and
- an active pharmaceutical ingredient, preferably an active pharmaceutical ingredient promoting the regeneration of biological tissue.

An antimicrobial agent can be added to prevent or treat infection when the semi-interpenetrating polymer network is used as a hydrogel in tissue regeneration, for example.

The semi-interpenetrating polymer network can also act as a carrier to deliver active pharmaceutical ingredients into the tissue. In the context of the present invention, examples of active pharmaceutical ingredients that promote the regeneration of biological tissue are: bone morphogenetic protein (BMP), platelet-derived growth factors (PDGF), transforming growth factor-Beta (TGF-beta), and parathyroid Hormone (PTH). Further in the context of the present invention, osteoconductive materials such as calcium phosphate ceramics are also regarded as suitable active pharmaceutical ingredients.

A second aspect of the first facet of the invention relates to a method for stabilising collagen in an aqueous environment at a threshold pH value of at least 5.0, preferably at least 6.0, more preferably at least 7.0. The method comprises the steps of:

a. adding a collagen stabiliser to an aqueous mixture of the collagen at a pH value below the threshold pH value, before
b. adjusting the pH value of the mixture to at least the threshold pH value.

The collagen is preferably native collagen, more preferably native type I collagen, most preferably native homogenised fibrillar type I collagen.
The collagen stabiliser is selected form the group consisting of:

- polysaccharides, preferably selected from the group consisting of alginate, carboxymethylcellulose, chitosan and glycosaminoglycans;
- gelatin; and
- poly-L-lactic acid.

The adjustment of the pH value in step b. is done by adding a base, preferably with sodium hydroxide. Preferably, the aqueous mixture comprises an aqueous buffer solution, preferably a phosphate buffer solution.
According to the second aspect of the present invention, after adding the collagen stabiliser, no covalent bond is formed between the collagen and the collagen stabiliser.
As described above, the choice of polymer network plays a subordinate role for the method of the first aspect of the invention. The inventors have even found that the invention is also effective completely independently of the cross-linkable polymer network.
The effects and advantages mentioned in relation to the first aspect of the invention with respect to the collagen, which becomes unstable at higher pH values, and the collagen stabiliser also apply to the second aspect of the invention.
The second aspect of the invention enables the use of collagen at higher pH values that are not normally suitable for unmodified collagen, the advantages of which can thus be utilised. As described above, unmodified collagen has a particularly high mechanical stability and a low immunogenic profile.
Of course, the collagen stabiliser can be selected in such a way that it has other specific advantages for the application in addition to stabilising the collagen. The skilled person is aware of the benefits and uses of the collagen stabiliser of the invention for biological purposes.
The stabilised mixture of collagen and the collagen stabiliser can have therapeutic purposes, as already mentioned above: Wound healing, bone grafts, drug delivery and tissue regeneration. The stabilised mixture can also have cosmetic uses such as skin rejuvenation, hair and nail growth, anti-aging creams and serums, lip fillers, dermal fillers, and moisturising skincare products.
The stabilised collagen obtained in the method of the second aspect of the invention can for example be incorporated in a polymer network of the first aspect of the invention. The problem of providing an improved method for producing a semi-interpenetrating polymer network is thus solved by the method of the second aspect of the invention.
In a preferred embodiment of the first or second aspect of the invention, the collagen stabiliser is cross-linked with itself. Additionally or alternatively, the collagen stabiliser is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, and acetylated potassium hyaluronate, preferably the type of hyaluronic acid is sodium hyaluronate;
- chondroitin sulfate;
- heparin; and
- heparan sulfate.

Hyaluronic acid is a naturally occurring glycosaminoglycan composed of repeating disaccharide units of D-glucuronic acid and N-acetyl-D-glucosamine, linked together by alternating beta-1,4 and beta-1,3 glycosidic bonds.
Chondroitin sulfate is a sulfated glycosaminoglycan composed of a chain of alternating sugars (N-acetylgalactosamine and glucuronic acid), with sulfate groups attached to the N-acetylgalactosamine sugar.
Heparin is a highly sulfated glycosaminoglycan and is the most negatively charged biological molecule. It is composed of variably sulfated repeating disaccharide units of uronic acid (either glucuronic acid or iduronic acid) and glucosamine. Heparan sulfate is structurally related to heparin but is less sulfated.
In the context of the present invention, a collagen stabiliser is cross-linked with itself if chemical bonds are formed between its molecular chains, resulting in a network where the chains are interconnected through covalent, ionic, or hydrogen bonds.
For example, hyaluronic acid (or any type thereof) can be cross-linked with itself by attaching thiols, hexadecylamides, and ty-ramines. It can also be cross-linked directly with formaldehyde or with divinylsulfone.
Preferably, the collagen stabiliser is sodium hyaluronate that is not cross-linked with itself.
Preferably, the collagen stabiliser has an intrinsic viscosity ranging from 1.5 m$^3$/kg to 5.5 m$^3$/kg, preferably

ranging from 2.0 m$^3$/kg to 5.0 m$^3$/kg, more preferably ranging from ranging from 2.5 m$^3$/kg to 4.5 m$^3$/kg.

It has been shown that these glycosaminoglycans are particularly suitable as collagen stabilisers. Especially hyaluronic acid (in various forms according to the invention, such as sodium hyaluronate) has proved to be particularly good in preventing collagen from aggregating and clumping at elevated pH values.

In the case of types of hyaluronic acid as collagen stabilisers, it is believed that the bioactivity of the collagen is enhanced by synergistic effects exerted by the type of hyaluronic acid.

In addition to their suitability as collagen stabilisers, the several types of hyaluronic acid have also been shown to act as viscosity modifiers. For the first aspect of the invention, this means that run-off of the semi-interpenetrating polymer network is prevented during application.

Preferably, for the first or second aspect of the invention, the molecular weight of the collagen stabiliser is at least 100 kDa (kilodalton), preferably at least 500 kDa, more preferably at least 1000 kDa, even more preferably at least 2000 kDa, most preferably at least 3000 kDa.

It was found that both collagen stabilisers with a comparatively low molecular weight (e.g. 500 kDa) and collagen stabilisers with a comparatively high molecular weight (i.e. high molecular weight collagen stabilisers; e.g. 2500 kDa) are suitable for stabilising collagen at elevated pH values.

The choice of molecular weight of the collagen stabiliser can therefore be selected in such a way that the rheological properties of the composition desired for the application can be achieved. For example, it is extremely advantageous for tissue regeneration applications if the composition remains inherently dimensionally stable and does not run until the in situ cross-linking reaction is complete. High molecular weight collagen stabilisers are therefore particularly advantageous for such applications, as they increase the viscosity of the composition. In the context of the present invention, a high molecular weight collagen stabiliser has a molecular weight of at least 1000 kDa, preferably at least 1500 kDa, more preferably at least 2000 kDa, most preferably at least 3000 kDa.

Mixtures of collagen and the collagen stabilisers, in particular types of hyaluronic acid, at a pH value of 7.0 demonstrated excellent long-term stability at temperatures of 25 °C, allowing long-term storage and transport.

A third aspect of the first facet of the invention relates to a semi-interpenetrating polymer network, preferably in the form of a hydrogel, obtainable by a method according to the first aspect of the invention.

The semi-interpenetrating polymer network, preferably in the form of a hydrogel, comprises an enclosed aqueous solution with a pH value of at least 5.0, preferably at least 6.0, even more preferably at least 7.0.

A fourth aspect of the first facet of the invention relates to a semi-interpenetrating polymer network, preferably in the form of a hydrogel, in particular the semi-interpenetrating polymer network of the third aspect of the invention, comprising:

- a covalently cross-linked polymer network, preferably a covalently cross-linked polyethylene glycol polymer network;
- collagen, preferably native collagen, more preferably native type I collagen, most preferably native homogenised fibrillar type I collagen;
- a collagen stabiliser selected from the group consisting of:

    - polysaccharides, preferably selected from the group consisting of alginate, carboxymethylcellulose, and glycosaminoglycans;
    - gelatin;
    - poly-L-lactic acid; and
    - chitosan;

- optionally, an aqueous buffer solution, preferably an aqueous phosphate buffer solution;
- optionally, a base, preferably sodium hydroxide, to neutralize the collagen to form non-charged collagen;
- optionally, an antimicrobial agent, preferably methyl-4-hydroxybenzoate and/or 1,1'-hexamethylenbis[5-(4-chlorphenyl)biguanid]; and
- optionally, an active pharmaceutical ingredient, preferably an active pharmaceutical ingredient promoting the regeneration of biological tissue.

The collagen and the collagen stabilizer are non-covalently embedded in the covalently cross-linked polymer network.

The problem of providing an improved method for producing a semi-interpenetrating polymer network is thus solved by the semi-interpenetrating polymer network of the third and fourth aspect of the invention.

In a preferred embodiment of the fourth aspect of the invention, the collagen stabiliser is cross-linked with itself.

Additionally or alternatively, the collagen stabiliser is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, and acetylated potassium hyaluronate, preferably the type of hyaluronic acid is sodium hyaluronate;
- chondroitin sulfate;
- heparin; and
- heparan sulfate.

A fifth aspect of the first facet of the invention relates to a kit of parts for the manufacture of a semi-interpenetrating polymer network, preferably in the form of a hydrogel. The kit of parts comprises:

- a first component comprising a first polymer comprising multiple nucleophilic functional groups, preferably a polymer comprising multiple thiol functional groups, more preferably a multifunctional polyethylene glycol thiol;
- a second component comprising a second polymer comprising multiple electrophilic functional groups, preferably a polymer comprising multiple $\alpha,\beta$-unsaturated carbonyl functional groups, preferably is a polymer comprising multiple acrylate functional groups, more preferably a multifunctional polyethylene glycol acrylate; and
- a set of further constituents comprising:

  - collagen, preferably native collagen, more preferably native type I collagen, most preferably native homogenised fibrillar type I collagen;
  - a collagen stabiliser selected from the group consisting of:

    - polysaccharides, preferably selected from the group consisting of alginate, carboxymethylcellulose, chitosan, and glycosaminoglycans;
    - gelatin; and
    - poly-L-lactic acid;

  - optionally, an aqueous buffer solution, preferably an aqueous phosphate buffer solution, or a dehydrated aqueous buffer solution, preferably a dehydrated aqueous phosphate buffer solution;
  - a base, preferably sodium hydroxide, to neutralize the collagen to form non-charged collagen;
  - optionally, an antimicrobial agent, preferably methyl-4-hydroxybenzoate and/or 1,1'-hexamethylenbis[5-(4-chlorphenyl)biguanid]; and
  - optionally, an active pharmaceutical ingredient, preferably an active pharmaceutical ingredient promoting the regeneration of biological tissue.

At least one of the first polymer and the second polymer is at least trifunctional.
In the context of the present invention, a component is a compound or a mixture that is stored separate from any other components which may be part of a kit until use. A use can mean that two components are mixed together and the resulting mixture is applied to a surface.
A component is stored in a container, for example in a syringe closed at the tip. The kit of parts may include other parts that enable the mixing of components and constituents and the application of the composition, for example onto a surface, into a gap or onto, between or into biological tissue. Other parts are for example Luer-couplers for syringes and/or adapters and/or static mixers.
The first component of the kit of parts according to the invention is devoid of the second polymer and the second component is devoid of the first polymer.
Mixing all components of the kit of parts of the fifth aspect of the invention results in an aqueous mixture with a pH value of at least 5.0, preferably at least 6.0, even more preferably at least 7.0.
The compounds and mixtures of the set of further constituents may be present in the first component, in the second component, in further components, or in a combination thereof.
In the context of the present invention, "a dehydrated aqueous buffer solution, preferably a dehydrated aqueous phosphate buffer solution" means that the components of the kit have been prepared by adding to at least one of the components an aqueous buffer solution and that the water content is removed from the component(s) comprising the aqueous buffer solution (e.g. by freeze-drying). Before use of the kit, the respective component(s) rehydrated by adding water. The water to be added can be supplied as part of the kit or there can be instructions on how much and what grade of water to use.
Any components of the kit comprising water can be supplied in dried form and rehydrated before use.
Removal of water from any of the components allows simpler sterilisation after preparing the components.
For example, a component is prepared using collagen, a collagen stabiliser, a base and an aqueous phosphate buffer solution. The combined mixture of the component is freeze-dried using a lyophiliser to remove the water content. The dried component is part of the kit. Also components comprising a first or second polymer as well as collagen, a collagen stabiliser, a base and an aqueous buffer solution can be provided in dried form in the kit, for example.

The kit of parts can be used to carry out the method of the first aspect of the invention and the kit of parts can be used to produce a semi-interpenetrating polymer network, preferably in the form of a hydrogel, of the third or fourth aspect of the invention.

The problem of providing an improved method for producing a semi-interpenetrating polymer network is thus solved by the kit of parts of the fifth aspect of the invention.

Preferably, the first polymer is at least tetrafunctional, more preferably is tetrafunctional. Preferably, the first polymer has a molecular weight in the range of 500 g/mol to 20000 g/mol, preferably in the range of 1000 g/mol to 10000 g/mol, more preferably in the range of 1500 g/mol to 5000 g/mol, even more preferably in the range of 1700 g/mol to 2300 g/mol most preferably in the range of 1900 g/mol to 2100 g/mol.

Preferably, the second polymer is at least tetrafunctional, preferably is tetrafunctional. Preferably, the second polymer has a molecular weight in the range of 500 g/mol to 20000 g/mol, preferably in the range of 1000 g/mol to 10000 g/mol, more preferably in the range of 1500 g/mol to 5000 g/mol, even more preferably in the range of 2000 g/mol to 2600 g/mol, most preferably in the range of 2200 g/mol to 2400 g/mol.

In a preferred embodiment of the fifth aspect of the invention, the collagen stabiliser is cross-linked with itself. Additionally or alternatively, the collagen stabiliser is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, and acetylated potassium hyaluronate, preferably the type of hyaluronic acid is sodium hyaluronate;
- chondroitin sulfate;
- heparin; and
- heparan sulfate.

In a preferred embodiment of the fifth aspect of the invention, each further constituent is contained in at least one of the first component, the second component, and (a) further component(s), and wherein

- the collagen is contained in a third and in a fourth component, and the collagen stabiliser is contained in the third and in the fourth component;
- the collagen is contained in the first and in the second component, and the collagen stabiliser is contained in the first and in the second component;
- the collagen is contained in a third component; and the collagen stabiliser, the optional aqueous buffer solution or the optional dehydrated aqueous buffer solution, and the base are contained in a fourth component; or
- the collagen and the collagen stabiliser are contained in a third component; and the optional aqueous buffer solution or the optional dehydrated aqueous buffer solution, and the base are contained in a fourth component.

It should be noted that the collagen without the collagen stabiliser should only be present in a component in an acidic aqueous environment, as the collagen becomes unstable at a pH value of at least 5.0, preferably at least 6.0, more preferably at least 7.0, and precipitates and/or forms aggregates. Only if the collagen is mixed with the collagen stabiliser may the pH value be raised above this value. This was discussed in detail in the previous aspects of the invention.

A sixth aspect of the first facet of the invention relates to the semi-interpenetrating polymer network of any one of the third or fourth aspect of the invention for use in the treatment of the human or animal body by surgery or therapy.

A seventh aspect of the first facet of the invention relates to the semi-interpenetrating polymer network of any one of the third or fourth aspect of the invention for use in a method for the regeneration and/or therapeutic augmentation of biological tissue.

The regeneration of biological tissue comprises skin regeneration, bone tissue engineering (e.g. guided bone regeneration, GBR), cartilage repair, neural tissue regeneration, corneal tissue regeneration, vascular tissue regeneration, and liver tissue regeneration.

Guided bone regeneration (GBR) is based on cell or tissue occlusive membranes. These GBR membranes allocate space for bone growth and prevent fast-proliferating soft tissues from competing with bone formation in the augmentate.

The therapeutic augmentation of biological tissue comprises cardiac tissue augmentation, spinal disc augmentation, soft tissue (e.g. muscle tissue or fat tissue) augmentation, vocal cord augmentation, bladder wall augmentation, urethral spincter augmentation, joint cartilage augmentation, and aortic wall augmentation.

An eighth aspect of the first facet of the invention relates to a method for the cosmetic treatment of biological tissue. The method comprises the steps:

- producing a composition comprising collagen and a collagen stabiliser in an aqueous environment at a pH value of at least 5.0, preferably at least 6.0, more preferably at least 7.0, according to the method of the second aspect of the invention; and

- injecting the composition into the biological tissue for cosmetic filling.

Alternatively, the method of the eight aspect of the invention comprises the step of:

- producing a semi-interpenetrating polymer network, preferably in the form of a hydrogel, according to the method of the first aspect of the invention, wherein the reaction between the first polymer and the second polymer is performed in situ in the biological tissue.

Preferably, the composition in both alternatives of the eight aspect is injected into a lip as a cosmetic lip filler, and/or the composition is injected into skin as a cosmetic dermal filler. In the second alternative, the cross-linking reaction takes place in the lip tissue and/or skin tissue.

As described in the preceding aspects of the invention, the collagen stabiliser allows chemically unmodified collagen to be used at higher pH values without precipitating and/or aggregating. The use of such unmodified collagen is particularly advantageous for cosmetic treatments such as lip fillers or dermal fillers.

A second facet of the invention closely related to the first facet of the invention concerns the production of a porous semi-interpenetrating polymer network, preferably in the form of a hydrogel.

Such porous hydrogels have similar characteristics as the semi-interpenetrating polymer networks described in the introductory paragraphs. Porous hydrogels are three-dimensional networks of polymer chains that are highly hydrated but can retain their structure due to physical or chemical crosslinking. The porosity refers to the presence of interconnected void spaces (pores) within the hydrogel matrix.

Their porous structure allows for loading and controlled release of drugs, making them ideal for targeted drug delivery systems. They can also be used as scaffolds for cell growth in tissue regeneration, aiding in the repair or replacement of damaged tissues or organs.

The use of phosphate ions has been described in WO 2023145765 A1 (UNIV TOKYO; GELLYCLE CO LTD, 03 August 2023) to induce the formation of aqueous two-phase systems (APTS) during in situ cross-linking PEG hydrogels, resulting in microporous hydrogels. The porosity is induced with relatively high phosphate ion concentrations (> 300 mM), potentially unfavourable to living systems, e.g. due to the high osmotic pressures they may exert. Also, the methods described in this document produce hydrogels with a relatively low viscosity and are too runny to allow for an adequate application in biological tissue, for example.

EP 3347061 B1 (ETH Zurich, 19 May 2021) describes the use of mixtures of hyaluronic acid and PEG to result in porous hydrogels. The described systems work at low PEG concentrations with very high reaction times and are considered unsuitable for an in situ application.

It is therefore an object of the second facet of the present invention to overcome at least some, if not all, of the shortcomings of the prior art. In particular, it is an object of the present invention to provide an improved method for producing a porous semi-interpenetrating polymer network.

The problem is solved by a method for producing a porous semi-interpenetrating polymer network, preferably in the form of a hydrogel; by a porous semi-interpenetrating polymer network; and by a kit of parts for the manufacture of a porous semi-interpenetrating polymer network.

Towards this end, a first aspect of the second facet of the invention pertains to a method for producing a porous semi-interpenetrating polymer network, preferably in the form of a porous hydrogel. This is done by reacting a first polymer with a second polymer to produce a covalently cross-linked polymer network in an aqueous environment in the presence of a porosifier composition. The porosifier composition comprises a type of hyaluronic acid in a mass fraction of 0.1 %-w/w to 10 %-w/w, preferably of 0.3 %-w/w to 5 %-w/w,, more preferably 0.5 %-w/w to 2 %-w/w with respect to the total mass of the porosifier composition; and phosphate ions, preferably as part of an aqueous phosphate buffer solution, in a concentration of 40 mM to 290 mM, preferably of 45 mM to 250 mM, more preferably 50 mM to 200 mM with respect to the total volume of the porosifier composition. At least one of the first polymer and the second polymer is at least trifunctional. In particular, the total volume fraction of the first polymer and the second polymer combined ranges from 3 %-v/v to 40 %-v/v, preferably ranges from 5 %-v/v to 30 %-v/v, more preferably ranges from 8 %-v/v to 20 %-v/v, most preferably ranges from 8 %-v/v to 16 %-v/v, with respect to the sum of volumes of constituents for producing the porous semi-interpenetrating polymer network.

In the context of the present invention, at least 5 %, preferably at least 10 %, more preferably at least 20 % of a cross-sectional area of a porous semi-interpenetrating polymer network consists of pores. Pores can be closed pores, open pores and pore interconnections, as described in EBRAHIMI, M. (Porosity parameters in biomaterial science: Definition, impact, and challenges in tissue engineering. Front. Mater. Sci. 2021, Vol. 15, No. 3, pages 352 to 373). At least 30 %, preferably at least 50 % of the open pores and closed pores has at least one spatial dimension, preferably at least two perpendicular spatial dimensions of at least 20 μm. The same definition is true for a porous hydrogel.

Specific types of hyaluronic acid, such as sodium hyaluronate, have been described above. The same specific types of hyaluronic acid also apply here.

By "the sum of volumes of constituents for producing the porous semi-interpenetrating polymer network" is meant the volume of the mixture directly after mixing the first polymer, the second polymer, the porosifier composition and any other ingredients, at the very beginning of the cross-linking reaction between the first polymer and the second polymer.

The description of the first polymer and second polymer and their specific embodiments have been sufficiently described above and also apply here.

The polymer concentration can be chosen to affect the properties of the resulting porous hydrogel. For example, the porous hydrogel is particularly stable if the volume fraction of the total polymer content ranges from 8 %-v/v to 20 %-v/v. A polymer content of 8 %-v/v to 16 %-v/v leads to interconnected pores. The cross-linking reaction leads to a phase separation, which results in the porosity of the end product. Without being bound to a theory, it is assumed that a large part of the type of hyaluronic acid and the phosphate ions separate from the polymer network and other possible constituents (e.g. collagen) during phase separation.

It has surprisingly been found that types of hyaluronic acid, in particular sodium hyaluronate, and phosphate ions exert a synergistic effect on the tendency of the mixtures to phase separate and form porous hydrogels.

This is particularly interesting because much higher phosphate ion concentrations are specified in prior art methods for the formation of porous hydrogels (WO 2023145765 A1, UNIV TOKYO; GELLYCLE CO LTD, 03 August 2023)). With the addition of a type of hyaluronic acid, the phosphate ion concentration can be significantly reduced, resulting in a much lower osmotic pressure. This is particularly advantageous in applications for the regeneration of biological tissue, in which a high osmotic pressure is unfavourable.

Conversely, due to the phosphate ions, phase separation and the formation of pores is also possible with a hyaluronic acid content as low as 2 %-w/w with respect to the mass of the porosifier composition, which is not indicated as possible in prior art processes (EP 3347061 B1 (ETH ZURICH, 19 May 2021). In contrast, it was found that mixtures comprising PEG (10 %-v/v with respect to the sum of volumes of constituents for producing the porous semi-interpenetrating polymer network) and 2 %-w/w of hyaluronic acid with respect to the porosifier composition did not induce phase separation if the phosphate ion concentration was below 25 mM, but at higher concentrations it did.

It was also found that the addition of phosphate ions and hyaluronic acid (or a type thereof) together had a greater effect on phase separation and the formation of pores than the sum of the individual constituents alone.

In a preferred embodiment of the first aspect of the second facet of the invention, the first polymer is a polyethylene glycol thiol; and/or the second polymer is a polyethylene glycol acrylate.

In another preferred embodiment of the first aspect of the second facet of the invention, the concentration of phosphate ions depends on the mass fraction of the type of hyaluronic acid. When the mass fraction of the type of hyaluronic acid ranges from 0.1 %-w/w to 1 %-w/w, preferably ranges from 0.5 %-w/w to 1 %-w/w, the concentration of phosphate ions ranges from 100 mM to 250 mM, preferably ranges from 100 mM to 200 mM. When the mass fraction of the type of hyaluronic acid ranges from 0.75 %-w/w to 1.25 %-w/w, the concentration of phosphate ions ranges from 75 mM to 250 mM, preferably ranges from 75 mM to 200 mM. And when the mass fraction of the type of hyaluronic acid ranges from 1.25 %-w/w to 3.00 %-w/w, preferably ranges from 1.25 %-w/w to 2.00 %-w/w, the concentration of phosphate ions ranges from 40 mM to 250 mM, preferably ranges from 50 mM to 200 mM. The mass fractions are indicated with respect to the total mass of the porosifier composition and the concentrations are indicated with respect to the total volume of the porosifier composition

Especially in these content combinations, a particularly good phase separation and pore formation is achieved.

In another preferred embodiment of the first aspect of the second facet of the invention, the first polymer is reacted with the second polymer at a temperature ranging from 18 °C to 40 °C, preferably at a temperature ranging from 22 °C to 38 °C, more preferably at a temperature ranging from 26 °C to 36 °C, most preferably at a temperature ranging from 30 °C to 34 °C.

The temperature can be modulated to further control pore formation. A higher temperature increases the tendency of mixtures to phase separate in the presence of phosphate ions and a type of hyaluronic acid.

A second aspect of the second facet of the invention relates to a porous semi-interpenetrating polymer network, preferably in the form of a porous hydrogel, obtainable by a method according to the first aspect of the second facet of the invention.

The resulting porous semi-interpenetrating polymer network, preferably in the form of a porous hydrogel, comprises a covalently cross-linked polymer network and the type of hyaluronic acid non-covalently embedded in the polymer network.

A third aspect of the second facet of the invention relates to a kit of parts for the manufacture of a porous semi-interpenetrating polymer matrix, preferably in the form of a porous hydrogel.

The kit of parts comprises: a first component comprising a first polymer comprising multiple nucleophilic functional groups, preferably a polyethylene glycol thiol. It also comprises a second component comprising a second polymer comprising multiple electrophilic functional groups, preferably a multifunctional polyethylene glycol acrylate. The kit further comprises a set of further constituents comprising: a type of hyaluronic acid; and phosphate ions, preferably as part of an aqueous phosphate buffer solution. The type of hyaluronic acid and the phosphate ions are present as or as part of a porosifier composition or can be combined to form a or part of a porosifier composition. The type of hyaluronic acid is present in a mass fraction of 0.1 %-w/w to 3 %-w/w, preferably of 0.5 %-w/w to 2 %-w/w, with respect to the total mass of the porosifier composition; and the phosphate ions, preferably as part of an aqueous phosphate buffer solution, are present in a concentration of 40 mM to 250 mM, preferably of 50 mM to 200 mM with respect to the total volume of the porosifier composition.

Preferably, the concentration of phosphate ions depends on the mass fraction of the type of hyaluronic acid, as described above.

The relevant, general parts of the description of the kit of parts, which has already been described above, also apply here.

The first component is devoid of the second polymer and the second component is devoid of the first polymer.

The further constituents (e.g. the type of hyaluronic acid and the phosphate ions) can for example be present in the kit premixed as the porosifier composition; or they can be present in components separate from each other. Also, they can be present in the first component and/or the second component.

The process, the porous semi-interpenetrating polymer network and the kit of parts can be used in the treatment of the human or animal body by surgery or therapy. They can find use in a method for the regeneration and/or therapeutic augmentation of biological tissue (e.g. guided bone regeneration). They can find use in a cosmetic method.

The invention will now be described by way of more specific embodiments. These embodiments are not intended to limit the gist of the invention in any way, but rather serve to ease understanding of the invention.

Fig. 1A & 1B: Native homogenised fibrillar type I collagen; A: Rheometric shear profile; B: differential interference contrast (DIC) micrograph (scale bar = 50 pm) ;

Fig. 2A - 2G: Neutralisation of collagen without a collagen stabiliser; A: photograph of the collagen suspension at various pH values; B & C: Rheometric amplitude sweep curves of 0.6 %-w/w collagen suspensions before (B) and after (C) neutralisation; D: DIC micrograph (scale bar = 100 pm); E - G: Amplitude sweep experiments of the collagen suspension before dilution and neutralisation (E), after dilution and before neutralisation (F), and after dilution and neutralisation (G);

Fig. 3A - 3I: Neutralisation of collagen with a collagen stabiliser (sodium hyaluronate); A: photograph of the mixture after centrifugation; B: DIC micrograph (scale bar = 200 pm); C-G: Rheological storage and loss moduli profiles from amplitude sweep tests of mixtures with varying amount fractions (shared Y-axis and legend); H & I: Amplitude sweep experiments before (H) and after (I) neutralisation;

Fig. 4A & 4B: Schematic illustrations of the stabilisation of collagen by a collagen stabiliser; A: Collagen hydrogels aggregate and sediment at a neutral pH value; B: intercalated collagen stabiliser prevents collagen from aggregating at higher pH values;

Fig. 5A - 5D: Range of possible molecular weights of the collagen stabiliser; A - C: frequency sweep experiments with varying molecular weights of the sodium hyaluronate used; D: complex viscosity curves with varying molecular weights of the sodium hyaluronate;

Fig. 6A - 6F: Fluorescence (A, C, E) and DIC Transmission (B, D, F) micrographs of semi-interpenetrating polymer networks comprising in situ cross-linked PEG polymers; A & B: PEG (12 %-w/w) with sodium hyaluronate (2 %-w/w), scale bars = 100 $\mu$m; C & D: PEG (12 %-w/w) with sodium hyaluronate (1.76 %-w/w) and collagen (0.26 %-w/w), scale bars = 100 $\mu$m; E & F: cutting face of the sample from C & D, scale bar E = 200 pm, scale bar F = 50 $\mu$m;

Fig. 7: Force distance compression curves comparing the elastic behaviour and maximum rupture forces of PEG polymer networks with embedded collagen and sodium hyaluronate; with only sodium hyaluronate embedded; and without any embedded biomacromolecule;

Fig. 8A - 8D: A & B: evaluation of gingiva fibroblast adhesion after 3 h, 6 h, 24 h, 48 h, and 72 h: negative control with a PEG-semi-interpenetrating polymer matrix with sodium hyaluronate (A), and PEG-semiinterpenetrating polymer matrix with sodium hyaluronate and collagen (B) (live imaging micrographs: scale bars = 100 $\mu$m; fluorescence micrographs, lower images after 24 h: scale bars = 50 pm); C & D:

Quantification of cell adhesion and proliferation after 72 hrs based on direct cell counts (C) and metabolic assays (MTT) (D) (NaHA = sodium hyaluronate; Col = collagen);

Fig. 9A - 9D: Porous semi-interpenetrating polymer networks; phase separation and pore formation as a function of phosphate ion and sodium hyaluronate content; A & B: photographs of samples incubated at room temperature (A) and at 32 °C (B); C & D: UV absorbance measurements at 600 nm for samples incubated at room temperature (C) and at 32 °C (D);

Fig. 10: Porous semi-interpenetrating polymer networks; fluorescence-microscopic evaluation of cut hydrogel samples as a function of phosphate ion and sodium hyaluronan content (scale bars = 500 pm);

Fig. 11: Porous semi-interpenetrating polymer networks; fluorescence-microscopic evaluation of PEG-hydrogels with 8 %-w/w, 10 %-w/w, 12 %-w/w, 16 %-w/w, and 20 %-w/w PEG mass fractions (scale bars: 8 %-w/w and 10 %-w/w: 100 $\mu$m; 12 %-w/w, 16 %-w/w, and 20 %-w/w: 200 $\mu$m).

In the following examples it is first shown how homogenised fibrillar collagen (1) suspensions, which are used for all subsequent examples, are prepared. Then it is shown how collagen (1) without collagen stabiliser (2) behaves in a neutral and basic medium. As a counter-example according to the invention, the stability improvement of the collagen (1) in presence of sodium hyaluronate as a collagen stabiliser (2) is shown. Also, the use of chondroitin sulfate as collagen stabiliser is (2) shown. Finally, an example of the production of a semi-interpenetrating polymer network with embedded collagen (1) and sodium hyaluronate is shown, as well as its properties.

Preparation of Homogenised Fibrillar Collagen Suspensions

The collagen (1) used for all examples is native homogenised fibrillar type I collagen. The native type I fibrillar collagen mass is homogenised by the following method.

The native type I fibrillar collagen can be obtained from purified bovine skin, for example, and is commercially available from the following manufacturers: Viscolma, Viscofan GmbH, Germany; Bovine Collagen Type I raw material, SYMATese, France; X-Pure, Rousselot, France.

2 g of collagen (1) mass (Viscolma, Viscofan GmbH, Germany, 10 %-w/w dry content with respect to the total collagen mass) were dispersed in 18 ml of demineralized water at room temperature. The pH value was determined using a pH meter and adjusted to pH 3 using glacial acetic acid or 1 M aqueous HCl, if required (see comment below). 40 mg of paraben was optionally added as an antimicrobial preservative. The resulting mixture was cooled to 0 °C using an ice bath (using a dry ice/isopropanol bath cooled to -20 °C is also an option leading to the same result) and dispersed using an Kinematica, Polytron PT 10-35 batch disperser equipped with a PTA 20S aggregate (Polytron Switzerland) operating at 12000 rpm for 30 seconds under ice cooling. The mixture was subsequently allowed to cool for 2 min (cooling for 1 min is also an option leading to the same result), and the dispersion-cooling sequence was repeated 2 times. The resulting gel-like suspension was transferred into a sterile syringe, the air was removed by centrifugation for 2 min at approximately 5300 rpm (approximately 7000 times the force of gravity (Haereus Sepatech, Megafuge 1.0; centrifugation for 5 min at approximately 3500 rpm (approximately 3500 times the force of gravity) is also an option leading to the same result), and the resulting gel was stored at 5-8°C. All operations were performed under sterile laminar flow and using sterilised equipment. A native homogenised fibrillar type I collagen suspension in water (1 %-w/w with respect to the total mass of the suspension) is obtained.

Collagen feedstock is available at varying pH values. The pH value only needs to be adjusted to 3, if it is above this value.

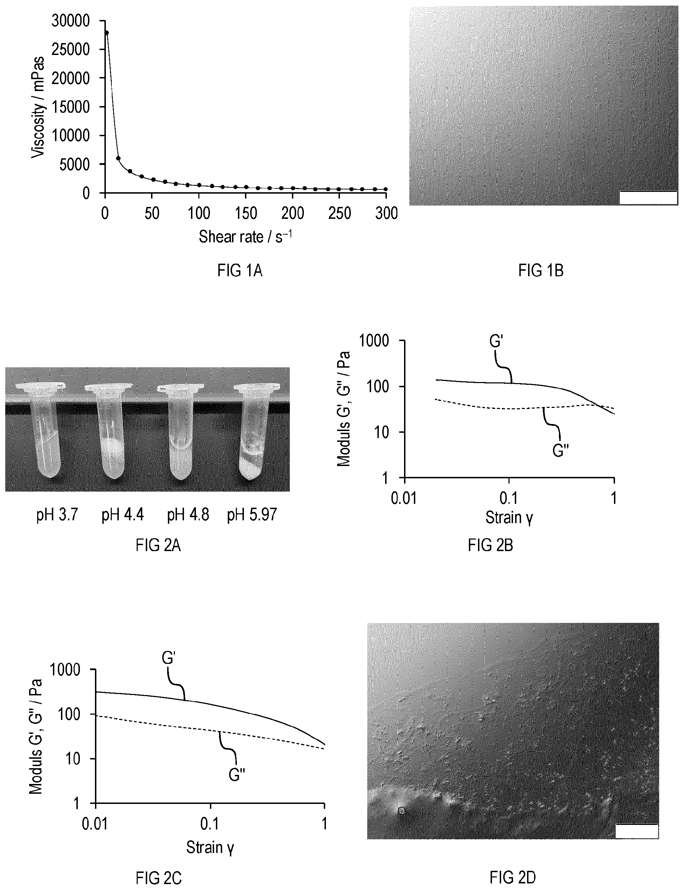

It is noted that the properties of the resulting homogenised fibrillar type I collagen suspension are highly dependent on factors such as the quantities used, the type of dispersing device used and the number of repetitions of the dispersion-cooling sequence. The process instruction described above provides a good starting point. However, it is advisable to adjust the parameters on this basis so that a rheometric shear profile as shown in Figure 1A is achieved (see description below). The viscosity should range from 19000 mPas to 28000 mPas at a shear rate of 2 s$^{-1}$; and from 400 mPas to 500 mPas at a shear rate of 300 s$^{-1}$.

Flow curve experiments (e.g. to characterise collagen (1) dispersions) were conducted using an Anton Paar MCR 52 (Anton Paar, Switzerland) using a cone-plate measuring system equipped with a CP25-1 cone with a diameter of 25 mm and an angle of 1°. Experiments were conducted at 25$\pm$0.01°C using the instruments Peltier unit for temperature control. The gels were pre-sheared at a shear rate of 1/s. Flow curve measurements were obtained using a gradient from 2/s to 300/s, with a resolution of 25 points per experiment and a measurement duration of 6 s per measuring time point.

Differential interference contrast (DIC) characterization was performed on an Olympus BX 61 (Olympus Japan) equipped with differential interference contrast Normarksi prism (Olympus U-DICT, Olympus Japan) and a digital camera (XC-30, Olympus Japan) using Olympus Cell-Sens imaging software. Collagen (1) substrates were diluted 10-100 fold in demineralised water acidified to ca. pH 3 using acetic acid, and single drops of the resulting suspension were applied on microscopy slides and air-dried.

Rheometric characterisation of the resulting native homogenised fibrillar type I collagen (1) suspension indicated a shear thinning profile: The associated high viscosities at low shear forces and low viscosities at high shear forces are considered ideal for application through syringes or applicators and stable retention at the application site (Figure 1A). Differential interference contrast (DIC) microscopic characterisation after diluting the suspension in water and drying the resulting mixture on a microscopy slide indicated that the suspension consisted of thin (ca. 1 $\mu$m thick) individual collagen (1) filaments with high suspension degree (Figure 1B). These filaments had a variable length of up to ca. 100 $\mu$m. The high dispersion of collagen (1) fibrils was considered ideal for achieving a homogeneous functionalisation of in situ cross-linking polymer networks.

Neutralisation of Collagen without a Collagen Stabiliser

The properties of the native homogenised fibrillar type I collagen (1) suspension, obtained as described in the previous section, as a function of an increasing pH value starting from approximately 3.7 were investigated.

The pH value of the collagen (1) suspension was gradually increased by adding increments of 10 $\mu$l of 0.1 N NaOH, stirring the resulting mixture with a spatula and measuring the pH with a pH-Meter (WTW 541 GLP equipped with a pH probe Sentix 41, Xylem Analytics GmbH, Germany).

Based on the data obtained from this titration, collagen (1) suspensions with different pH values of 3.7 (original native homogenised type I collagen suspension), 4.4, 4.8 and 5.97 were prepared by adding corresponding equivalents of NaOH. The resulting mixtures were stirred using a spatula, and the mixtures were centrifuged at approximately 3000 rpm for 1 min (approximately 500 times the force of gravity) to test the mixtures for syneresis.

Figure 2A shows a photograph of these collagen (1) suspensions. At a pH value of 3.7 and 4.4, the suspensions showed a homogeneous distribution of the collagen (1). The sample at a pH value of 4.8 showed first indications of phase separation and the sample at a pH value of 5.97 showed a strong degree of phase separation and sedimentation of the previously suspended collagen (1). It seems as if the collagen (1) hydrogel collapses at a pH value of 5 or higher.

Rheological storage and loss moduli measurements were conducted using an Anton Paar MCR 52 (Anton Paar, Switzerland). The software used for the data acquisition and evaluation for all measurements of this patent application using this device was "Anton Paar Rheocompass" (Version 1.24.259, Anton Paar, Austria).

Amplitude strain sweep experiments were performed using an Antoon Paar MCR 52 Rheometer (Anton Paar MCR 52 Rheometer) with a cone-plate measuring system equipped with a CP50-1 Cone plate (Diameter 49.965 mm, Cone Angle (0.997°). Experiments were carried out at 25±0.01 °C using the instrument Peltier unit for temperature control. The gap size within the cone plate setup during experiments was 0.100 mm. Experiments were started using 1 min of waiting time after sample application and lowering the cone to the final measuring distance for structure recovery. Samples were tested at an oscillating shear deformation with a constant angular frequency of $\omega$=10 rad/s and an increasing logarithmic shear deformation ramp ranging from 0.1% to 100 %. The resolution of the experiments was 50 points per experiment using the instrument's automatic default settings to determine the duration per measuring time point.

Figures 2B and 2C show the storage (G') and loss (G") moduli (i.e. rheological amplitude sweep curves) of 0.6 %-w/w native homogenised type I collagen (1) suspension at a pH value of 3.7 (thus, before increasing the pH value) and at a pH value of 8.0, respectively. The data indicate that the gels become much stiffer and weaker upon neutralisation, which can be associated with the aggregation, clumping up and sedimentation of collagen (1) fibres upon neutralization as visualized by the DIC images in Figure 2D. It was also found that neutralisation mainly affected the storage modulus of the gel, which was mainly associated with the disintegration of the collagen (1) fibre network with increasing pH values of the suspensions.

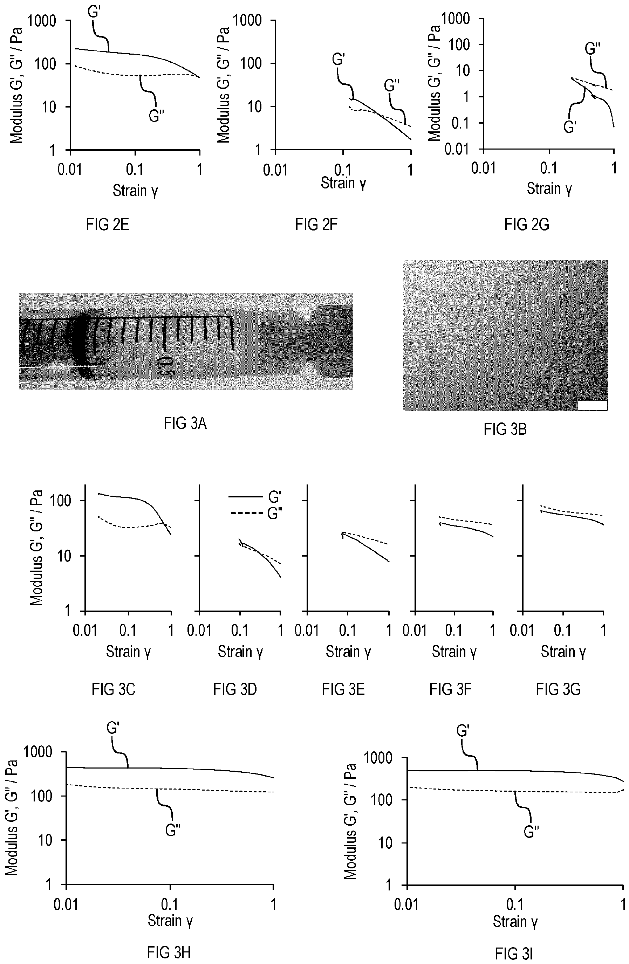

Figure 2E shows the amplitude sweep profile of a 0.6 %-w/w fibrillar collagen (1) suspension at a pH value of 3.0 indicating a mostly rigid and solid-like rather than fluid structure, higher storage moduli than loss moduli, and a decrease of the structural integrity of the gel at a strain ($\gamma$; length ratio, dimensionless) of above 30 %.

Figure 2F shows the amplitude sweep profile of the same collagen (1) suspension as in Figure 2E diluted to a 0.3 %-w/w collagen (1) content. At this collagen (1) content, storage and loss moduli were much lower than at a higher collagen (1) content. At low strain, the gel displayed a rigid and solid structure; and increasing the strain led to a loss of rigidity and transition to a fluid-like system. This is well in line with a diluted, still reticulated network of collagen (1) fibres.

The amplitude sweep profile of the collagen (1) suspension from Figure 2F after neutralisation is shown in Figure 2G. The loss and storage moduli are very low in comparison indicating phase separation of the collagen (1) suspension and sedimentation.

Hydrogels of Collagen and Sodium Hyaluronate as Collagen Stabiliser

As a collagen stabiliser (2), sodium hyaluronate as a type of hyaluronic acid was used to stabilise the collagen (1) at elevated pH values (above 5.0).

The sodium hyaluronate used for this experiments is commercially available under the brand name Hyatrue (Bloomage Biotechnology Corporation Ltd., China; HA EP3.8). It is not cross-linked with itself and has an intrinsic viscosity of 3.4 $m^3$/kg to 4.5 $m^3$/kg ($M_w$ = approximately 3000 kDa). Alternatively, such sodium hyaluronate can be obtained from Altergon (Italy). Alternatively, Hyatrue (Bloomage Biotechnology Corporation Ltd., China; HA EP3.0) with an intrinsic viscosity of 2.7 $m^3$/kg to 3.6 $m^3$/kg ($M_w$ = approximately 2600 kDa) can be used.

The molecular weight ($M_w$) can be approximated with the intrinsic viscosity according to the Mark-Houwink-Sakurada equation:

$$\eta = \kappa M_w^\alpha \qquad\qquad (\text{Equation 1})$$

wherein $\eta$ is the intrinsic viscosity, and $\kappa$ as well as the exponent $\alpha$ are specific for the polymer molecule and the solvent. According to Laurent et al. (Fractionation of Hyaluronic Acid. The Polydispersity of Hyaluronic Acid from the Bovine Vitreous Body. Biochim. Biophys. Acta. 1960, Vol. 42, pages 476 to 485), in the case of hyaluronic acid or sodium hyaluronate in an aqueous medium, $\kappa$ is 0.000036 and $\alpha$ is 0.78.

A native homogenised type I collagen (1) suspension (0.6 %-w/w in demineralised water, pH 3.0) was prepared; and a sodium hyaluronate gel (2 %-w/w in 50 mM phosphate buffer, pH 7.04) was prepared. The suspension and the gel were mixed at a 1:1 volume ratio and the pH value of the resulting mixture was adjusted to 7.0.

As illustrated in Figure 3A, the resulting neutralised Col/HA mixtures have a slightly opaque to transparent appearance and are stable upon centrifugation with a homogeneous consistency. The DIC micrograph in Figure 3B further indicates the presence of highly dispersed collagen (1) fibres in the gel.

This finding is in line with the rheological profile measurements. Figures 3C and 3G show the rheological storage and loss moduli profiles of the collagen (1) suspension alone and the sodium hyaluronate gel alone, respectively. Given the generally higher storage than loss moduli and the relatively steep decline of the storage moduli, the profile of collagen (Figure 3C) shows that the collagen (1) suspension comprises a relatively strong, but elastic interconnected network of collagen (1) fibrils that becomes disrupted at high shear forces. The profile of the sodium hyaluronate gel (Figure 3G) shows relatively high loss and storage moduli even at a high oscillation stress, indicating relatively strong but viscous structure of the sodium hyaluronate gels.

Figures 3D, 3E and 3F show the storage and loss moduli profiles of neutralised mixtures between the collagen (1) suspension and the sodium hyaluronate gel at volume ratios of 2:8, 5:5 and 8:2, respectively. They indicate that rheological profiles of mixtures of collagen (1) and sodium hyaluronate were mainly governed by a viscous rather than elastic behaviour, indicating that these mixtures were also characterised by a relatively weak network structure that flows freely upon application of oscillatory stresses. This result indicates that from a molecular view, high molecular weight chains from the collagen stabiliser, which are negatively charged at neutral pH, may intercalate and possibly form a complex with the overall neutral collagen fibrils and prevent such fibrils from aggregating and precipitating. Thus, the rheological profiles support the observed stabilisation of high molecular weight HA on neutralised fibrillar collagen (1) suspensions.

Amplitude sweep profiles of a collagen (1) (0.3 %-w/w) and sodium hyaluronate (2 %-w/w) suspension at a pH value of 3.0 (Figure 3H) and after neutralisation (Figure 3I) were also recorded. The loss and storage moduli before and after neutralisation are very similar. This indicates that structural changes in the gels remained marginal upon neutralisation, as opposed to the significant changes observed for the pure collagen (1) gels (see Figures 2F and 2G as a comparison). These findings corroborate the assumption that collagen (1) fibres are well dispersed in the sodium hyaluronate matrix, preventing individual collagen (1) fibres from aggregating and sedimenting. Further, both samples displayed a wide linear viscoelastic region (LVER) indicative of a well-dispersed and stable system. The comparison of the samples comprising both collagen (1) and sodium hyaluronate (Figures 3H and 3I) with samples comprising collagen (1) only (Figures 2F and 2G) also show that the high storage and loss moduli in the samples comprising both collagen (1) and sodium hyaluronate are governed by the presence of the sodium hyaluronate.

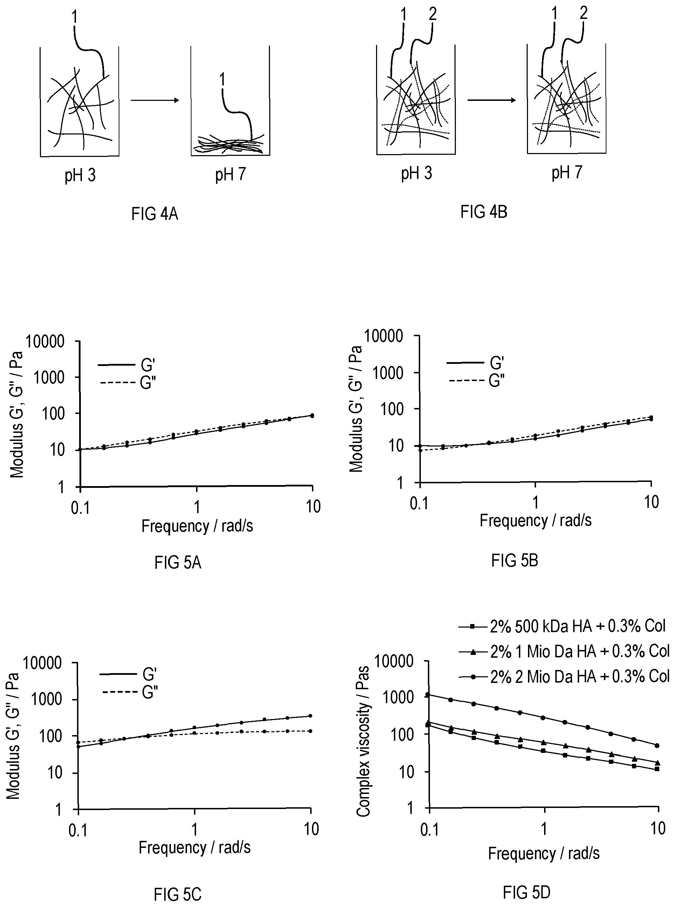

The stabilising effect of the collagen stabiliser (2) is illustrated in Figures 4A and 4B. Without wishing to be bound by any theory, collagen (1) hydrogels are stabilised by electrostatic repulsion between individual negatively charged collagen (1) fibres at low pH values. When the pH value is increased, the collagen (1) fibres become net-non-charged and thus lose the electrostatic repulsion resulting in aggregation and sedimentation of the collagen (1) fibres (Figure 4A). The intercalation of a collagen stabiliser (2), such as sodium hyaluronate, prevents the collagen (1) fibres from aggregating.

It was found that mixtures of collagen (1) (0.3 %-w/w) and sodium hyaluronate (2 %-w/w) are stable over long time periods at a pH value of 7.0 and 25 °C. Even after a storage period of 4 months, no signs of decomposition were detected by measuring the rheometric amplitude sweep profile (loss and storage moduli).

Range of Possible Molecular Weights of the Collagen Stabiliser

Various samples of sodium hyaluronate with different molecular weights were tested as collagen stabilisers (2).

The sodium hyaluronate types used were:

- Shandong 400-600 cosmetic grade; Shandong Focuschem Biotech Co. Ltd., Shandong, China, approx. 500 kDa (400 kDa to 600 kDa) ;
- Axenic, pharmaceutical grade; Amazon purchase, Axenic, Australia; approx. 1100 kDa; and
- Bloomage HA-E3.0; Bloomage Biotechnology Co. Ltd., China; approx. 2300 kDa.

Frequency sweep experiments were performed using an Anton Paar MCR 52 Rheometer (Anton Paar MCR 52 Rheometer; the same software was used as described above) with a cone-plate measuring system equipped with a CP 50-1 cone plate. Experiments were carried out at $25\pm0.01$ °C using the instrument's Peltier unit for temperature control. The gap size within the cone plate setup during experiments was 0.100 mm. Experiments were started using 1 min of waiting time after sample application and lowering the cone to the final measuring distance for structure recovery. Samples were tested at a constant oscillating shear deformation of 5 % after verifying the deformation within the LVMR. The angular frequency ($\omega$) was increased logarithmically from 0.1 rad/s to 100 rad/s. The resolution of the experiments was 16 points per experiment using the instrument's automatic default settings to determine the duration per measuring time point.

Figures 5A, 5B and 5C show the data for frequency sweep measurements of neutralised mixtures of collagen (1) (0.3 %-w/w) and sodium hyaluronate (2 %-w/w) with molecular weights of the sodium hyaluronate of 500 kDa (Figure 5A), 1100 kDa (Figure 5B), and 2300 kDa (Figure 5C).

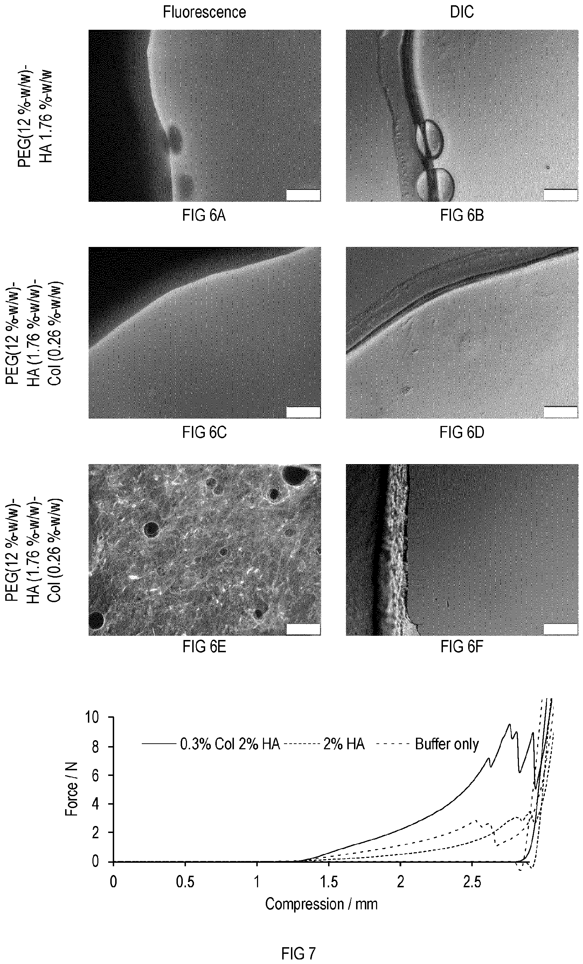

Figure 6 shows the comparison of the complex viscosities of the same three mixtures with the sodium hyaluronate with a different molecular weight.

These data support the presence of a well-structured (gelled) system in all Col/HA mixtures irrespective of the molecular weight of the sodium hyaluronate. Mixtures with 1100 kDa and 2300 kDa sodium hyaluronate display profiles that are particularly characteristic of rigid and associated structures with storage modulus (G') greater than the loss modulus (G") and independent of frequency. Such profiles are characteristic of stable systems in which sedimentation is unlikely to occur during storage.

This shows that the collagen stabiliser (2) can be used with a wide range of molecular weights, conserving the structural properties of the mixtures.

Chondroitin Sulfate as a Collagen Stabiliser

Chondroitin sulfate was investigated as a further collagen stabiliser (2).

The chondroitin sulfate used was ScanDroitin (Bovine Chondroitin Sulfate Sodium, Ph. Eur., Lot FV00753, ZPD AS, Denmark). Chondroitin sulfate was dissolved in demineralised water to obtain an aqueous 20 %-w/w chondroitin sulfate solution, using a laboratory stirrer at room temperature.

A homogenised fibrillar collagen (1) suspension (0.6 %-w/w) was prepared as described above.

NaOH (60 $\mu$l, 0.1 M; the amount needed to neutralise the homogenised fibrillar collagen (1) suspension) was added to 1 ml of the chondroitin sulfate solution and the solution was then combined with 1 ml of the collagen (1) suspension in a manner described above in the collagen (1) and sodium hyaluronate examples. The resulting gel mixture was centrifuged in a syringe at 5200 rpm for 3 min to remove air and to test for possible phase separation and sedimentation.

DIC microscopic analysis showed that no phase separation takes place and thus also no sedimentation was observed. The collagen (1) fibres appeared well dispersed within the mixture, suggesting that chondroitin sulfate acts as a collagen stabiliser (2).

Semi-interpenetrating polymer networks

These examples describe the preparation of a semi-interpenetrating polymer network in the form of a hydrogel in which a PEG polymer network is cross-linked in situ and in which collagen (1) and sodium hyaluronate as collagen stabiliser (2) are embedded.

Semi-interpenetrating polymer network - Example 1

Firstly, a stabilised mixture of collagen (1) and sodium hyaluronate is prepared with a pH value of about 7.5. A 4-arm PEG star-shaped polymer with four acrylate functional groups at the end of each arm (approx. 2300 g/mol) is combined with the stabilised mixture to produce a PEG-acrylate/collagen/sodium hyaluronate pre-mixture. Similarly, a 4-armed PEG star-shaped polymer with four thiol functional groups at the end of each arm (approx. 2000 g/mol) is combined with the stabilised mixture to produce a PEG-thiol/collagen/sodium hyaluronate pre-mixture. Mixing the two pre-mixtures initiates the in situ cross-linking reaction between the acrylate and the thiol functional groups yielding a semi-interpenetrating polymer network as a hydrogel.

The following paragraphs show the experimental details.

An aqueous phosphate buffer solution (50 mM, pH 7.52) was prepared by dissolving $K_2HPO_4$ (726 mg) and $KH_2PO_4$ (113 mg) in $H_2O$ (100 ml) .

The aqueous buffer solution and the sodium hyaluronate were steam-sterilised (121 °C, 15 min) in a laboratory steam sterilizer (W&H, Lisa 517 Sterilizer) before preparing a mixture of sodium hyaluronate (400 mg, Hyatrue, HA EP3.8, intrinsic viscosity 3.4 - 4.5 m³/kg, Bloomage, China) in the aqueous phosphate buffer solution (10 ml, 50 mM, pH 7.52) using two syringes connected using a female-to-female Luer coupling piece. The resulting mixture was allowed to swell overnight at room temperature, resulting in a 4 %-w/w sodium hyaluronate aqueous gel (HA/PO$_4$//4 %-w/w, HMW, EP3.8/50 mM, pH 7.5); HMW = high-molecular weight.

A native homogenised fibrillar type I collagen (1) suspension (0.6 %-w/w with respect to the total mass of the suspension) was prepared according to the same procedure as described above with the following changes: No acid was added, as the pH value of the collagen feedstock was already at a pH value of approximately 3. 1.2 g collagen (1) mass (Viscolma, Viscofan GmbH, Germany, 10 %-w/w dry content) and 18.8 ml demineralised $H_2O$ were used. The centrifugation to remove air was done for 5 min at 3500 rpm. The density of the resulting mixture is approximately 1 g/cm³.

An aqueous solution of NaOH (540 pg, 0.1 M) was added to the 4 %-w/w sodium hyaluronate aqueous gel (10 ml, HA/PO$_4$//4 %-w/w, HMW, EP3.8/50 mM, pH 7.5) and the resulting mixture was combined with the 0.6 %-w/w native homogenised fibrillar type I collagen (1) suspension (10 ml) using two syringes connected using a female-to-female Luer coupling piece. Mixing was accomplished by back-and-forth transfer between individual syringes. Handling was performed under a sterile laminar flow at room temperature. The resulting pH-neutral phosphate-buffered collagen/sodium hyaluronate-mixture (HA/Col/PO$_4$ // 2 %-w/w, HMW, EP3.8/0.3 %-w/w/25 mM, pH 7.5) was stored at room temperature. The density of the resulting mixture is approximately 1 g/cm³.

A PEG-acrylate/collagen/sodium hyaluronate pre-mixture was prepared by combining a PEG-acrylate (60 mg, 2300 g/mol, 4-armed star-shaped, tetrafunctional) with the pH-neutral phosphate-buffered collagen/sodium hyaluronate-mixture (440 mg, 25 mM, pH 7.5). Similarly, a PEG-thiol/collagen/sodium hyaluronate pre-mixture was prepared by combining a PEG-thiol (60 mg, 2000 g/mol, 4-armed star-shaped, tetrafunctional) with the pH-neutral phosphate-buffered collagen/sodium hyaluronate-mixture (440 mg, 25 mM, pH 7.5). The resulting pre-mixtures contained a 12 %-w/w PEG content with respect to the total mass of the respective pre-mixture. Again, mixing was performed using two syringes connected using a female-to-female Luer coupling piece by back-and-forth transfer between individual syringes.

Mixing of the resulting PEG-acrylate/collagen/sodium hyaluronate and PEG-thiol/collagen/sodium hyaluronate premixtures was performed in Luer-coupled sterile 2 mL syringes by back and forward movement (20 - 30 times). Alternatively, mixing can also be done by using a commercially available mixing dual barrel syringe equipped with a static mixer (e.g. Mixpack, Switzerland or DXM, Korea).

The content of each of the two PEG polymers is 6 %-w/w with respect to the total mass of the composition, resulting in a total PEG content of 12 %-w/w.

The content of sodium hyaluronate in the final composition is approximately 1.76 %-w/w, and the content of collagen (1) approximately 0.26 %-w/w, with respect to the total mass of the composition.

The pre-mixtures were used without further delay and mixed at room temperature or simulated application temperatures, i.e. 32 °C simulating oral applications, or 37 °C for medical applications at body temperature.

Semi-interpenetrating Polymer Network - Example 2

Depending on the application of the semi-interpenetrating polymer network, the quantities of the ingredients can be adjusted, for example to adapt the reaction time of the cross-linking reaction or the hardness of the network.

In example 2, the same process steps were carried out with the same quantities of the same ingredients as in example 1, with the following differences:

An aqueous phosphate buffer solution (400 mM, pH 7.00) was prepared by combining $K_2HPO_4$ (4641 mg) and $KH_2PO_4$ (1817 mg) in $H_2O$ (100 ml) .

The sodium hyaluronate aqueous gel (4 %-w/w with respect to the total mass of the gel) was prepared according to the same procedure as described in Example 1 with the following changes: Sodium hyaluronate (400 mg, Hyatrue, HA EP3.0, intrinsic viscosity 2.7 - 3.6 m³/kg, Bloomage, China; instead of HA EP3.8 from Example 1) was combined with aqueous phosphate buffer solution (10 ml, 400 mM, pH 7.00) to produce a sodium hyaluronate aqueous gel (HA/PO$_4$//4 %-w/w, HMW, EP3.0/400 mM, pH 7.0).

A native homogenised fibrillar type I collagen (1) suspension (1.0 %-w/w with respect to the total mass of the suspension) was prepared according to the same procedure as described in Example 1 with the following changes: 2.0 g collagen (1) mass (Viscolma, Viscofan GmbH, Germany, 10 %-w/w dry content) and 18.0 ml demineralised $H_2O$ were added.

An aqueous solution of NaOH (900 pg, 0.1 M) was added to the 4 %-w/w sodium hyaluronate aqueous gel (10 ml, $HA/PO_4//4$ %-w/w, HMW, EP3.0/400 mM, pH 7.0) and the resulting mixture was combined with the 1.0 %-w/w native homogenised fibrillar type I collagen (1) suspension (10 ml) according to the method described in Example 1. The resulting pH-neutral phosphate-buffered collagen/sodium hyaluronate-mixture ($HA/Col/PO_4$ // 2 %-w/w, HMW, EP3.0/0.5 %-w/w/200 mM, pH 7.0) was stored at room temperature.

The preparation of the PEG-acrylate/collagen/sodium hyaluronate and the PEG-thiol/collagen/sodium hyaluronate pre-mixtures was prepared in the same way and with the same PEG amounts as in Example 1, but at a temperature of 32 °C.

The content of sodium hyaluronate in the final composition is approximately 1.8 %-w/w, and the content of collagen (1) approximately 0.44 %-w/w, with respect to the total mass of the composition.

Fluorescence and DIC Transmission Microscopy

Fluorescence microscopy was performed on an Olympus IX 60 equipped with an Olympus XC 30 digital camera and operating with a filter cube Olympus U-MF2 and CellSense as an image recording and processing software (all Olympus, Japan). The microscope was equipped with an EXFO X-Cite 10 PC light source (EXFO, Canada).

Figure 6 shows fluorescence (A, C, E) and DIC Transmission (B, D, F) micrographs of Rhodamine-stained semi-interpenetrating polymer networks comprising in situ cross-linked PEG polymers (Example 1). Figures 6A and 6B show a PEG polymer network (12 %-w/w) with sodium hyaluronate (1.76 %-w/w) and Figures 6C and 6D show the same composition, additionally with collagen (1) (0.26 %-w/w). Figures 6A to 6D show the border region of the hydrogels. These Figures indicate that the hydrogels consist of one continuous homogenous phase without pores or inhomogeneities, demonstrating their suitability as cell-barrier material, for example. Figures 6E and 6F show fluorescence micrographs of the gel bulk phase and DIC micrographs of the cutting face of the hydrogel comprising PEG, sodium hyaluronate and collagen (1), respectively. Both micrographs evidenced the homogenous dispersion of collagen (1) fibres throughout the gel.

Viscosity in Relation to the Collagen Stabiliser's Concentration

It was found that increasing the content of the collagen stabiliser (2), in particular in the case of sodium hyaluronate, resulted in stiffer and stronger hydrogels. Even a minor change from 1 %-w/w to 2 %-w/w of sodium hyaluronate content with respect to the total mass of the semi-interpenetrating polymer network hydrogel improved the run-off behaviour of the hydrogel, keeping it form-stable during the cross-linking reaction.

Cross-linking Polymerisation Rate as a Function of the pH Value

To determine the reaction time of the PEG cross-linking reaction, rheometric isothermic polymerisation kinetics were performed on an Anton Paar MCR 52 using a cone-plate measuring system equipped with a CP25-1 cone with a diameter of 25 mm and an angle of 1°. Experiments were conducted using a gap distance of 1.5 mm at a temperature of 32 °C and by operating an oscillating shear deformation $\gamma$ of 0.1 % at a constant frequency of 1 Hz and using a data recording interval of 2 s. Experiments were typically conducted by starting the experiments without samples and injecting the samples between the plates through a static mixer with the time point of injection defining the start of the experiment. The polymerisation time was assessed by determining the sol-gel point where the storage modulus (G') became equal to or greater than the loss modulus (G").

It was found that a pH value of 7.5 and a temperature of 32 °C leads to a cross-linking reaction time of approximately 3 min for the PEG polymer networks from Examples 1 and 2 above. At a pH of 8.6, the reaction time is shorter with approximately 50 s. These durations and durations in-between are optimal for most applications of such hydrogels.

At lower pH values, the reaction time would be too long, which shows how important it is to make the collagen (1) stable at higher pH values using the collagen stabiliser (2).

Mechanical Properties of the Semi-interpenetrating Polymer Networks

Mechanical compression tests of semi-interpenetrating polymer networks and corresponding reference hydrogels were performed on a texture analyzer (TA XT plus, Sable Micro Systems operated using Exponent Software, Version 6.1.16) in compression mode at the ambient temperature (25 °C). Cylindrical Hydrogel samples Ø8 × 1 mm) were placed on the stationary platform and compressed by a moving probe (Ø6 mm stainless steel cylinder). The force applied as a function of distance was recorded at a rate of 0.02 s per data point. Compression test was conducted with a low loading velocity of 0.20 mm/s. The starting distance was set to 3 mm above the platform, and the endpoint of measurement was set to 2000 g, respectively.

Figure 7 shows the mechanical properties of PEG polymer networks with embedded collagen (1) prepared from collagen hyaluronate mixtures containing collagen (0.3 %-w/w) and sodium hyaluronate (2 %-w/w) (prepared with a method according to the invention), with only sodium hyaluronate, prepared using a phosphate buffered hyaluronate gel (2 %-w/w), and without any embedded biom-acromolecule using only buffer. It shows that the collagen (1) functionalisation renders the hydrogels mechanically significantly more resistant. Higher mechanical resistance, as indicated by the higher maximum rupture forces (vertical lines), supports the model that collagen (1) fibres are homogeneously distributed throughout the PEG matrix, forming a semi-interpenetrating polymer network with collagen (1) fibres reinforcing the mechanical strength of the network.

Degradation Properties as a Function of the PEG Concentration

In vitro degradation experiments were performed by a modified procedure reported earlier by Wechsler et al. (A novel, tissue occlusive poly(ethylene glycol) hydrogel material. J. Biomed. Mater. Res. 2008, Vol. 85A, No. 2, pages 285 to 292) by preparing corresponding PEG hydrogels from stabilized mixtures containing sodium hyaluronate (2 %-w/w) and collagen (1) (0.3 %-w/w) as 1 mm thin sheets in a custom-made Teflon cast at 37 °C. Hydrogels were allowed to polymerise for 15 min before stamping out disks of 6 mm diameter. Individual discs were incubated in 5 ml of 30 mM PBS ($1 \times$ PBS; phosphate-buffered saline, pH 7.4) buffer at 37 °C. Weighing the samples after careful removal of excess adhering liquid using a dry tissue and replacement of incubation buffer with new buffer was performed in 1-week intervals.

Hydrogels were prepared with a total PEG content (the first and second PEG content together) of 5 %-w/w, 10 %-w/w, and 15 %-w/w with respect to the total mass of the hydrogel, according to the procedures described in Examples 1 and 2.

The samples with 5 %-w/w, 10 %-w/w, and 15 %-w/w total PEG content had a degradation time of 30 days, 50 days, and 60 days, respectively. This shows that the degradation time can easily be adapted based on the application by modulating the PEG content.

Comparison of the degradation time of PEG polymer networks with and without collagen (1) and a collagen stabiliser (2) (e.g. sodium hyaluronate) also showed that the degradation time is not impacted by the addition of collagen (1), sodium hyaluronate (or any other collagen stabiliser (2)), or the combination thereof.

Cell Adhesion of the Semi-interpenetrating Polymer Networks

In vitro assays were carried out in 12 or 24 multiwell culture plates coated with 100 $\mu$l of corresponding in situ cross-linked hydrogels under a laminar flow cabinet before use. Substrates were stored at 2 °C to 8 °C until use to prevent drying. All samples were based on a total amount of PEG of 12 %-w/w, comprising equal amounts of 4-arm-PEG thiol and 4-arm PEG acrylate (as described above). Negative controls (Sample 1) were prepared by combining 60 $\mu$l of the PEG-acrylate and 60 $\mu$l PEG-thiol separately, with 440 $\mu$l of an autoclaved (15 min, 121 °C) 1 %-w/w high molecular weight sodium hyaluronate (intrinsic viscosity 2.7-3.2 $m^3$/kg, Altergon, Italy) in 25 mM Phosphate buffer, pH 7.0. The resulting PEG acrylate/sodium hyaluronate and PEG thiol/sodium hyaluronate mixtures were subsequently combined by mixing in luer-coupled syringes before applying the resulting ISC hydrogel mixtures as thin layers in corresponding 12 or 24-well-tissue culture polystyrene plates. Test samples (Sample 2) were prepared according to the procedure described for Sample 1 but by using a collagen/sodium hyaluronate mixtures containing approximately 0.3 %-w/w Col and approximately 1 %-w/w HA prepared according to the procedures described above instead of the sodium hyaluronate-component of Sample 1. Formulations for cell experiments did not contain Paraben.

In vitro assays were performed using primary gingiva-derived fibroblasts isolated from healthy extracted third molars and cultured in Dulbecco's Modified Eagle's Medium (DMEM) with 10% fetal calf serum (FCS, Sigma-Aldrich, St.Louis, MO, USA) and 1%PenStrep (Thermo Fisher Scientific, Waltham, MA, USA). For cell experiments, the hydrogels were either seeded with 50000 cells for imaging analysis (12 multiwell culture plates) or with 20000 cells for their quantification (24 multiwell culture plates). Cells were initially seeded in DMEM/1XPenStrep without serum to avoid interference of the latter with the adhering process. After 3h, non-adherent cells were removed by gentle washing with phosphate-buffered saline (PBS) solution and refed with complete culturing medium (DMEM/10%FCS/1XPenStrep).

Morphological analysis of cell adhesion was performed by live imaging and immunofluorescence (IF). Live imaging pictures of cells grown on ISC hydrogels and controls were taken 3, 6 and 24h after seeding using an inverted microscope (Leica Microsystems, Wetzlar, Germany). For IF, 24h after seeding, cells were washed twice with PBS before fixation in 4%PFA for 20 min at RT. Cells were then washed 3X with PBS, permeabilized in 0.1% Triton-X-100 for 5min and rinsed three times with PBS prior to incubation with a primary polyclonal anti-fibronectin antibody for 2h at RT in 3% bovine serum albumin (BSA) solution. Afterwards, cells were extensively rinsed in PBS and incubated with fluorescent-labelled secondary goat anti-rabbit antibody (Molecular Probes, Thermo Fisher Scientific) with tetramethylrhodamine (TRITC)-phalloidine (Sigma-Aldrich) for 1h at RT in the dark. Finally, cells were washed 3X in PBS and once with ddH2O before being coverslip-mounted. Samples were examined under an Olympus BX-51 phase/fluorescence microscope (Olympus Life Science Solutions) equipped for fluorescence.

Quantification of adhered cells was performed by direct counting of the cells or by assessing cell metabolic activity after 72h with an MTT (3-(4,5-dimethylthiazol-2-yl)-2,5-diphenyltetrazolium bromide) assay (Sigma-Aldrich). Assays were performed directly in the cell culture plates. Furthermore, the number of adhered cells was monitored over time (24, 48 and 72 hours) by live imaging microscopy. For direct cell counting, cells were trypsinized using a 0.25% Trypsin/EDTA solution and manually counted using a Neubauer counting chamber device. Otherwise, for the measuring of the metabolic activity, cells were incubated with the MTT solution at a final concentration of 0.5mg/ml for 4h to allow MTT conversion into formazan in metabolically active cells. After two rinses with PBS, formazan crystals were solubilized with a 4N HCl solution. Sample absorbance was therefore read at 570nm on an EL808 BioTek microplate reader (BioTek, Winooski, VT, USA).

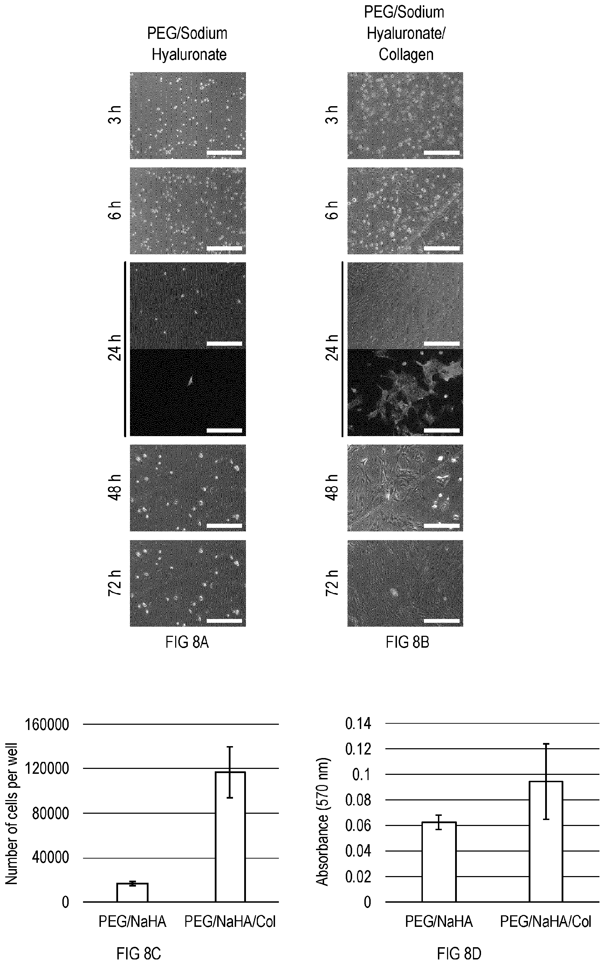

Morphological analysis of cell adhesion is shown in Figures 8A and 8B. Both the sample without (Sample 1, Figure 8A) and the sample with (Sample 2, Figure 8B) collagen led to some initial cell adhesion. However, only PEG-sodium hyaluronate/collagen containing 1 %-w/w sodium hyaluronate and 0.3 %-w/w collagen (Sample 2) resulted in significant cell adhesion, spreading and proliferation on the substrate.

Staining for the cytoskeleton (actin, red, Figures 8A and 8B, lower image after 24 h) and for the fibroblast marker fibronectin (green) after 24 h confirmed that fibroblast appeared well spread on sample 2 (with collagen), while adhesion or spreading was limited or widely absent on sample 1 (without collagen).

The images acquired after 24 h, 48 h and 72 h further demonstrated that gingiva fibroblasts adhered and proliferated well on PEG-sodium hyaluronate/collagen test samples. Negative control PEG-hydrogels (sample 1) showed very little amounts of attached and spread cells after 72 hrs.

The quantification of adhered cells after 72 hours, shown in Figures 8C and 8D, confirms the abilities of PEG-collagen/sodium hyaluronate hydrogels with a concentration of 0.3 %-w/w collagen to promote cell adhesion and proliferation. Specifically, a significantly higher number of cells were detected on PEG-sodium hyaluronate/collagen test substrates (Sample 2) compared to negative controls (Sample 1) both from direct cell counts (Figure 8C) as well as from metabolic assays based on 3-(4,5-dimethyl-thiazol-2-yl)-2,5-diphenyltetrazolium bromide (MTT) (Figure 8D).

Porous Semi-interpenetrating Polymer Networks

For synthesising porous semi-interpenetrating polymer networks, the same 4-armed star-shaped PEG polymers were used as described in section "Semi-interpenetrating polymer network - Example 1."

The sodium hyaluronate used was "Hyatrue HA EP3.0" (intrinsic viscosity 2.7 - 3.6 $m^3$/kg, approx. 2300 kDa, Bloomage Biotechnology Co. Ltd., China).

Sodium hyaluronate was dissolved in demineralised water to prepare 2 %-w/w and 4 %-w/w stock solutions at room temperature. The resulting gels were allowed to swell overnight to result in homogeneous mixtures. Mixing and dissolving of sodium hyaluronate in water was accomplished in coupled Luer syringes.

An aqueous phosphate buffer stock solution using $K_2HPO_4$ and $KH_2PO_4$ with a $PO_4^{3-}$ concentration of 400 mM and a pH value of 7 was prepared.

From the sodium hyaluronate and buffer stock solutions, 20 porosifier compositions (1 ml each) were prepared comprising all combinations which cover the following contents with respect to the total mass or volume of the porosifier composition:

- sodium hyaluronate: 0 %-w/w, 0.5 %-w/w, 1.0 %-w/w, and 2.0 %-w/w; and
- phosphate ions: 25 mM, 50 mM, 75 mM, 100 mM, and 200 mM.

The porosifier compositions were prepared using Luer-coupled syringes as mixing devices.

Equal volumes of the 4-armed star-shaped PEG-thiol (first polymer) and 4-armed star-shaped PEG-acrylate (second polymer) were added to the porosifier compositions to obtain a total volume fraction of the first polymer and the second polymer combined of 10 %-v/v, with respect to the sum of volumes of constituents for producing the porous semi-interpenetrating polymer network.

After thorough mixing, 0.5 ml of each mixture was immediately transferred to a well of a well plate. The mixtures were incubated at room temperature or at 32 °C. In the context of the present invention, room temperature is a temperature ranging from 18 °C to 28 °C, preferably ranging from 21 °C to 25 °C, more preferably of approximately 23 °C.

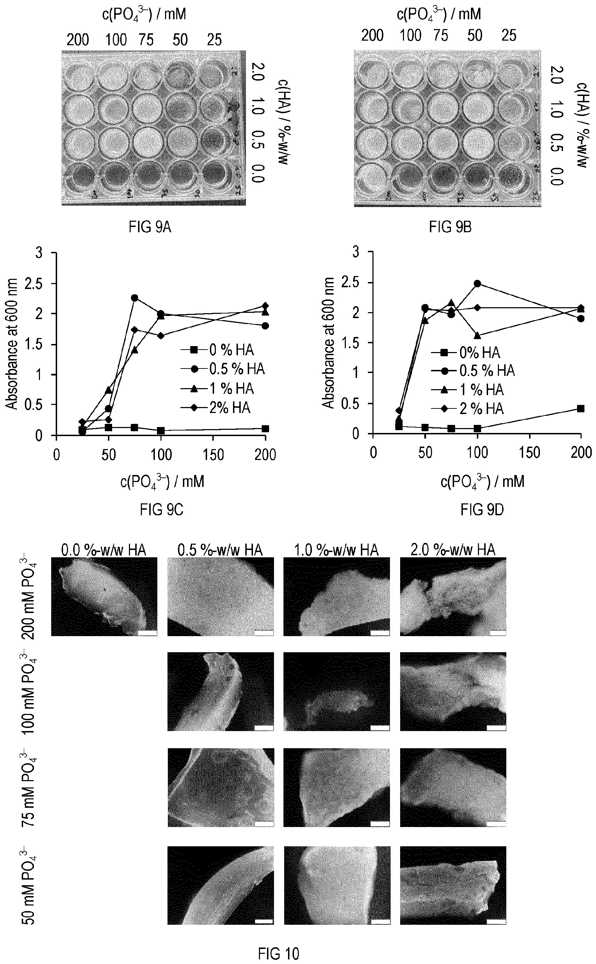

Figures 9A and 9B illustrate the appearance of the hydrogels after polymerisation at room temperature (A) and at 32 °C (B), respectively. Samples polymerised at room temperature in pure buffer without sodium hyaluronate were all transparent at both temperatures. Samples polymerised at room temperature at low phosphate concentrations (25 mM) were transparent, irrespective of their sodium hyaluronate content, indicating the inability of phosphate ion concentrations or sodium hyaluronate contents in the tested ranges to induce phase separation alone. Room temperature samples with low phosphate ion concentrations (50 mM) and containing 0.5 %-w/w to 2 %-w/w sodium hyaluronate appeared slightly opaque. Samples containing sodium hyaluronate with phosphate concentrations equal to 75 mmol or greater appeared opaque and untransparent, indicating strong phase separation.

Samples polymerized at 32 °C (Figure 9B) showed similar behaviour, but the tendency of mixtures containing 50 mmol phosphate and 0.5 %-w/w to 2 %-w/w sodium hyaluronate to phase separate and result in opaque samples appeared to increase. Furthermore, samples containing no sodium hyaluronate and a 200 mM phosphate ion concentration appeared to result in slightly porous gels at 32 °C, compared to samples that were polymerised at room temperature and remained fully transparent. These observations indicate that temperature may influence the gels' phase diagram, promoting phase separation at higher temperatures. However, an increase in temperature alone without the addition of sodium hyaluronate does not lead to sufficient pore formation and the resulting hydrogels are too runny.

UV-absorbance measurements were performed by mounting the well plates into a UV-VIS spectrometer beam and manually adjusting for the correct position and orientation in the beam. Measurements were performed at 600 nm, and the obtained results are a measure of turbidity, i.e. phase separation of and within the samples, respectively.

The data from the UV-absorbance measurements in Figures 9C (incubation at room temperature) and 9D (incubation at 32 °C) as a measure of opaqueness are consistent with the visual observations made in Figures 9A and 9B.

To study the phase separation and tendency of PEG hydrogels to phase separate and form macropores as a function of phosphate ion and sodium hyaluronate concentrations, the as-prepared hydrogel samples were investigated using fluorescence microscopy.

Fluorescence microscopy was performed as described above. Hydrogels were fluorescence stained by adding 5 $\mu$l or 10 $\mu$l of a 10 mg/ml ethanol Rhodamine B solution per well. Gels were cut into thin slices and transferred to a microscopy glass slide. Clear and transparent samples (i.e. all samples with a phosphate ion concentration of 25 mM; and the samples with a phosphate ion concentration of below 200 mM and a sodium hyaluronate content of 0 %-w/w) were not considered for microscopic evaluation.

The results of this investigation and the qualitative evaluation of the tendency of the formulations to phase separate and/or form macropores are shown in Figure 10 and Table 1, respectively. Specifically, the qualitative evaluation indicated that only samples with higher sodium hyaluronate and phosphate ion concentrations displayed both, i.e. a phase separation and a tendency to form macropores. Samples prepared at intermediate phosphate ion and/or sodium hyaluronate concentrations, e.g. 50 mM phosphate ion and 0.5 %-w/w sodium hyaluronate as well as 200 mM phosphate ion and 0 %-w/w sodium hyaluronate, displayed phase separation without forming a macroporous structure. Specifically, these samples displayed an inhomogeneous PEG phase without showing the formation of microscopically visible macropores.

In the context of the present invention a macropore is a pore with at least one spatial dimension, preferably with at least two perpendicular spatial dimensions, of at least 100 pm.

A micropore is a pore with at least one spatial dimension, preferably with at least two perpendicular spatial dimensions, of at least 1 $\mu$m and smaller than 100 pm.

Table 1: Qualitative evaluation of porosity and phase separation derived from fluorescence micrographs; 0: Optically transparent, not evaluated; 1: Phase separation, no macropores; 2: Phase separation, macropores

| PO$_4^{3-}$ concentration | Sodium hyaluronate content | | | |
| --- | --- | --- | --- | --- |
| | 0 %-w/w | 0.5 %-w/w | 1.0 %-w/w | 2.0 %-w/w |
| 200 mM | 1 | 2 | 2 | 2 |
| 100 mM | 0 | 2 | 2 | 2 |
| 75 mM | 0 | 1 | 2 | 2 |
| 50 mM | 0 | 1 | 1 | 2 |
| 25 mM | 0 | 0 | 0 | 0 |

Porosity as a function of the PEG concentration

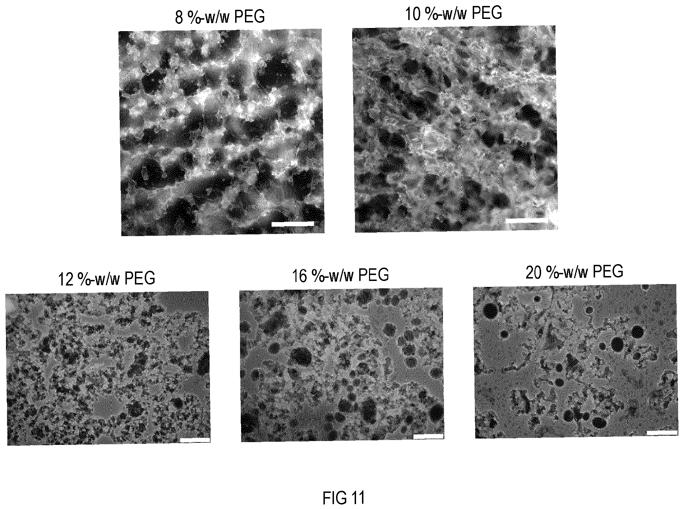

Figure 11 shows fluorescence micrographs of porous semi-interpenetrating polymer networks (porous hydrogels) with different mass fractions of the PEG polymer network of 8 %-w/w, 10 %-w/w, 12 %-w/w, 16 %-w/w and 20 %-w/w with respect to the total mass of the porous semi-interpenetrating polymer network. The same sodium hyaluronate as described above was used. The porous hydrogels were prepared from a stock solution with a sodium hyaluronate content of 1 %-w/w and a phosphate ion concentration of 200 mM. The PEG polymers were added to this stock solution according to the method as described above. The cross-linking reaction was performed at 32 °C between microscopy glass slides to evaluate the resulting pore structures.

The data in Figure 11 show that low PEG concentrations result in relatively open PEG-networks. Loosely reticulated aggregates of PEG characterise porous hydrogels with a PEG content of 8 %-w/w. Intermediate PEG concentrations result in well-coalesced structures with an open porous structure with open interconnected pores. PEG concentrations of 16 %-w/w result in gels that show a combination of open porous regions and regions that display isolated and closed pores. Porous hydrogels with a 20 %-w/w PEG content appear to be still porous.

The porous hydrogels display a complex and hierarchically organised macro- (at least 100 pm) and micropore (at least 1 $\mu$m and smaller than 100 pm) topography with pore sizes spanning several length scales. Such systems are ideal candidates for regenerative applications.

**Claims**

1. A method for producing a semi-interpenetrating polymer network, preferably in the form of a hydrogel, by reacting a first polymer comprising multiple nucleophilic functional groups with a second polymer comprising multiple electrophilic functional groups to produce a covalently cross-linked polymer network in an aqueous environment at a pH value of at least 5.0, preferably at least 6.0, more preferably at least 7.0 in the presence of

   - collagen (1), preferably native collagen, more preferably native type I collagen, most preferably native homogenised fibrillar type I collagen; and
   - a collagen stabiliser (2) selected from the group consisting of:

      - polysaccharides, preferably selected from the group consisting of alginate, carboxymethylcellulose, chitosan and glycosaminoglycans;
      - gelatin; and
      - poly-L-lactic acid;

   wherein

      - at least one of the first polymer and the second polymer is at least trifunctional; and
      - the collagen (1) and the collagen stabiliser (2) get non-covalently embedded in the covalently cross-linked polymer network to produce the semi-interpenetrating polymer network.

2. The method of claim 1, wherein, before the first polymer is reacted with the second polymer,

      - a stabilised mixture with a threshold pH value of at least 5.0, preferably at least 6.0, more preferably at least 7.0 is prepared by

         - combining the collagen (1) with the collagen stabiliser (2) at a pH value below the threshold pH value, before adding a base, preferably sodium hydroxide, and optionally, an aqueous buffer solution, preferably an aqueous phosphate buffer solution; or
         - combining the collagen stabiliser (2) with a base, preferably sodium hydroxide, and optionally, an aqueous buffer solution, preferably an aqueous phosphate buffer solution, before adding the collagen (1); and

      - optionally, at least a part of the stabilised mixture is combined with the first polymer and/or at least a part of the stabilised mixture is combined with the second polymer.

3. The method of any one of the preceding claims, wherein the first polymer is reacted with the second polymer at a temperature ranging from 22 °C to 47 °C, preferably at a temperature ranging from 27 °C to 42 °C, more preferably at a temperature ranging from 32 °C to 37 °C.

4. The method of any one of the preceding claims, wherein the first polymer comprising multiple nucleophilic functional groups

      - is a polymer comprising multiple thiol functional groups, preferably is a multifunctional polyethylene glycol thiol;
      - is at least tetrafunctional, preferably is tetrafunctional;
      - has a molecular weight in the range of 500 g/mol to 20000 g/mol, preferably in the range of 1000 g/mol to 10000 g/mol, more preferably in the range of 1500 g/mol to 5000 g/mol, even more preferably in the range of 1700 g/mol to 2300 g/mol most preferably in the range of 1900 g/mol to 2100 g/mol; or

- fulfils a combination of these conditions.

5. The method of any one of the preceding claims, wherein the second polymer comprising multiple electrophilic functional groups

- is a polymer comprising multiple $\alpha,\beta$-unsaturated carbonyl functional groups, preferably is a polymer comprising multiple acrylate functional groups, more preferably is a multifunctional polyethylene glycol acrylate;
- is at least tetrafunctional, preferably is tetrafunctional;
- has a molecular weight in the range of 500 g/mol to 20000 g/mol, preferably in the range of 1000 g/mol to 10000 g/mol, more preferably in the range of 1500 g/mol to 5000 g/mol, even more preferably in the range of 2000 g/mol to 2600 g/mol, most preferably in the range of 2200 g/mol to 2400 g/mol; or
- fulfils a combination of these conditions.

6. The method of any one of the preceding claims, wherein the reaction between the first polymer and the second polymer is carried out in the presence of at least one further constituent selected from the group consisting of:

- an antimicrobial agent, preferably methyl-4-hydroxybenzoate and/or 1,1'-hexamethylenbis[5-(4-chlorphenyl) bi-guanid];
- an aqueous buffer solution, preferably an aqueous phosphate buffer solution;
- a base, preferably sodium hydroxide, to neutralize the collagen to form non-charged collagen; and
- an active pharmaceutical ingredient, preferably an active pharmaceutical ingredient promoting the regeneration of biological tissue.

7. A method for stabilising collagen (1) in an aqueous environment at a threshold pH value of at least 5.0, preferably at least 6.0, more preferably at least 7.0, the method comprising the steps of:

- adding a collagen stabiliser (2) to an aqueous mixture of the collagen (1) at a pH value below the threshold pH value, before
- adjusting the pH value of the mixture to at least the threshold pH value;

wherein

- the collagen (1) is preferably native collagen, more preferably native type I collagen, most preferably native homogenised fibrillar type I collagen; and
- the collagen stabiliser (2) is selected form the group consisting of:

  - polysaccharides, preferably selected from the group consisting of alginate, carboxymethylcellulose, chitosan and glycosaminoglycans;
  - gelatin; and
  - poly-L-lactic acid.

8. A method of any one of the preceding claims, wherein the collagen stabiliser (2) is cross-linked with itself and/or is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, and acetylated potassium hyaluronate, preferably the type of hyaluronic acid is sodium hyaluronate;
- chondroitin sulfate;
- heparin; and
- heparan sulfate.

9. A semi-interpenetrating polymer network, preferably in the form of a hydrogel, obtainable by a method according to any one of claims 1 to 6 or 8.

10. A semi-interpenetrating polymer network, preferably in the form of a hydrogel, in particular the semi-interpenetrating polymer network of claim 9, comprising:

- a covalently cross-linked polymer network, preferably a covalently cross-linked polyethylene glycol polymer network;
- collagen (1), preferably native collagen, more preferably native type I collagen, most preferably native homogenised fibrillar type I collagen;
- a collagen stabiliser (2) selected from the group consisting of:

- polysaccharides, preferably selected from the group consisting of alginate, carboxymethylcellulose, and glycosaminoglycans;
- gelatin;
- poly-L-lactic acid; and
- chitosan;
- optionally, an aqueous buffer solution, preferably an aqueous phosphate buffer solution;
- optionally, a base, preferably sodium hydroxide, to neutralize the collagen to form non-charged collagen;
- optionally, an antimicrobial agent, preferably methyl-4-hydroxybenzoate and/or 1,1'-hexamethylenbis[5-(4-chlorphenyl)biguanid]; and
- optionally, an active pharmaceutical ingredient, preferably an active pharmaceutical ingredient promoting the regeneration of biological tissue;

wherein the collagen (1) and the collagen stabiliser (2) are non-covalently embedded in the covalently cross-linked polymer network.

11. The semi-interpenetrating polymer network of claim 10, wherein the collagen stabiliser (2) is cross-linked with itself and/or is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, and acetylated potassium hyaluronate, preferably the type of hyaluronic acid is sodium hyaluronate;
- chondroitin sulfate;
- heparin; and
- heparan sulfate.

12. A kit of parts for the manufacture of a semi-interpenetrating polymer network, preferably in the form of a hydrogel, the kit of parts comprising:

- a first component comprising a first polymer comprising multiple nucleophilic functional groups, preferably a polymer comprising multiple thiol functional groups, more preferably a multifunctional polyethylene glycol thiol;
- a second component comprising a second polymer comprising multiple electrophilic functional groups, preferably a polymer comprising multiple $\alpha,\beta$-unsaturated carbonyl functional groups, preferably is a polymer comprising multiple acrylate functional groups, more preferably a multifunctional polyethylene glycol acrylate; and
- a set of further constituents comprising:

- collagen (1), preferably native collagen, more preferably native type I collagen, most preferably native homogenised fibrillar type I collagen;
- a collagen stabiliser (2) selected from the group consisting of:

- polysaccharides, preferably selected from the group consisting of alginate, carboxymethylcellulose, chitosan and glycosaminoglycans;
- gelatin; and
- poly-L-lactic acid;

- optionally, an aqueous buffer solution, preferably an aqueous phosphate buffer solution, or a dehydrated aqueous buffer solution, preferably a dehydrated aqueous phosphate buffer solution;
- a base, preferably sodium hydroxide, to neutralize the collagen to form non-charged collagen;
- optionally, an antimicrobial agent, preferably methyl-4-hydroxybenzoate and/or 1,1'-hexameth-ylenbis[5-(4-chlorphenyl)biguanid]; and
- optionally, an active pharmaceutical ingredient, preferably an active pharmaceutical ingredient promoting the regeneration of biological tissue;

wherein at least one of the first polymer and the second polymer is at least trifunctional.

13. The kit of parts of claim 12, wherein the collagen stabiliser (2) is cross-linked with itself and/or is a glycosaminoglycan selected from the group consisting of:

- a type of hyaluronic acid selected from the group consisting of hyaluronic acid, sodium hyaluronate, potassium hyaluronate, hydrolysed hyaluronic acid, acetylated sodium hyaluronate, acetylated potassium hyaluronate, preferably the type of hyaluronic acid is sodium hyaluronate;
- chondroitin sulfate;
- heparin; and
- heparan sulfate.

14. The kit of parts of any one of claims 12 and 13, wherein each further constituent is contained in at least one of the first component, the second component, and (a) further component(s), and wherein

- the collagen (1) is contained in a third and in a fourth component, and the collagen stabiliser (2) is contained in the third and in the fourth component;
- the collagen (1) is contained in the first and in the second component, and the collagen stabiliser (2) is contained in the first and in the second component;
- the collagen (1) is contained in a third component; and the collagen stabiliser (2), the optional aqueous buffer solution or the optional dehydrated aqueous buffer solution, and the base are contained in a fourth component; or
- the collagen (1) and the collagen stabiliser (2) are contained in a third component; and the optional aqueous buffer solution or the optional dehydrated aqueous buffer solution, and the base are contained in a fourth component.

15. The semi-interpenetrating polymer network of any one of claims 9 to 11 for use in the treatment of the human or animal body by surgery or therapy.

16. The semi-interpenetrating polymer network of any one of claims 9 to 11 for use in a method for the regeneration and/or therapeutic augmentation of biological tissue.

FIG 1A

FIG 1B

pH 3.7    pH 4.4    pH 4.8    pH 5.97

FIG 2A

FIG 2B

FIG 2C

FIG 2D

FIG 2E

FIG 2F

FIG 2G

FIG 3A

FIG 3B

FIG 3C

FIG 3D

FIG 3E

FIG 3F

FIG 3G

FIG 3H

FIG 3I

FIG 4A

FIG 4B

FIG 5A

FIG 5B

FIG 5C

FIG 5D

FIG 6A — FIG 6B — FIG 6C — FIG 6D — FIG 6E — FIG 6F

FIG 7

PEG/Sodium
Hyaluronate

3 h

6 h

24 h

48 h

72 h

FIG 8A

PEG/Sodium
Hyaluronate/
Collagen

3 h

6 h

24 h

48 h

72 h

FIG 8B

Number of cells per well

160000

120000

80000

40000

0

PEG/NaHA    PEG/NaHA/Col

FIG 8C

Absorbance (570 nm)

0.14

0.12

0.1

0.08

0.06

0.04

0.02

0

PEG/NaHA    PEG/NaHA/Col

FIG 8D

c(PO$_4^{3-}$) / mM

200  100  75  50  25

c(HA) / %-w/w

2.0  1.0  0.5  0.0

FIG 9A

c(PO$_4^{3-}$) / mM

200  100  75  50  25

c(HA) / %-w/w

2.0  1.0  0.5  0.0

FIG 9B

- 0 % HA
- 0.5 % HA
- 1 % HA
- 2% HA

FIG 9C

- 0% HA
- 0.5 % HA
- 1 % HA
- 2 % HA

FIG 9D

0.0 %-w/w HA    0.5 %-w/w HA    1.0 %-w/w HA    2.0 %-w/w HA

200 mM PO$_4^{3-}$    100 mM PO$_4^{3-}$    75 mM PO$_4^{3-}$    50 mM PO$_4^{3-}$

FIG 10

8 %-w/w PEG

10 %-w/w PEG

12 %-w/w PEG

16 %-w/w PEG

20 %-w/w PEG

FIG 11

Europäisches
Patentamt

European
Patent Office

Office européen
des brevets

## EUROPEAN SEARCH REPORT

Application Number

EP 24 16 2814

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BURKE K. CHAN ET AL: "Robust and Semi-Interpenetrating Hydrogels from Poly(ethylene glycol) and Collagen for Elastomeric Tissue Scaffolds", MACROMOLECULAR BIOSCIENCE, vol. 12, no. 11, 1 November 2012 (2012-11-01), pages 1490-1501, XP055236436, DE ISSN: 1616-5187, DOI: 10.1002/mabi.201200234 * abstract * * page 1492, paragraph 2.3 * ----- | 1-6,9-16 | INV. A61L27/44 |
| X | YAN MINGYAN ET AL: "Preparation of self-assembled collagen fibrillar gel from tilapia skin and its formation in presence of acidic polysaccharides", CARBOHYDRATE POLYMERS, vol. 233, 1 April 2020 (2020-04-01), page 115831, XP093193917, GB ISSN: 0144-8617, DOI: 10.1016/j.carbpol.2020.115831 * abstract * * page 2, paragraph 2.4 * * pages 4-5, paragraph 3.2.1 * * figure 2 * ----- -/-- | 7,8 | TECHNICAL FIELDS SEARCHED (IPC) A61L |

The present search report has been drawn up for all claims

| Place of search | Date of completion of the search | Examiner |
|---|---|---|
| The Hague | 9 August 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CUNDALL R B ET AL: "Polyelectrolyte complexes. 2. Interaction between collagen and polyanions", INTERNATIONAL JOURNAL OF BIOLOGICAL MACROMOLECULES, ELSEVIER BV, NL, vol. 1, no. 5, 1 December 1979 (1979-12-01), pages 215-222, XP023450072, ISSN: 0141-8130, DOI: 10.1016/0141-8130(79)90016-3 [retrieved on 1979-12-01] * abstract * * page 217, column 1 * * tables 3, 4 * * figures 4-6 * | 1-16 | |
| X | US 2010/055184 A1 (ZEITELS STEVEN M [US] ET AL) 4 March 2010 (2010-03-04) * claims 1, 16, 39 * | 1-6,9-16 | |
| | | | TECHNICAL FIELDS SEARCHED (IPC) |

The present search report has been drawn up for all claims

| Place of search | Date of completion of the search | Examiner |
|---|---|---|
| The Hague | 9 August 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 16 2814

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2024

| Patent document cited in search report | Publication date | Patent family member(s) | | Publication date |
|---|---|---|---|---|
| US 2010055184 A1 | 04-03-2010 | EP | 2341953 A2 | 13-07-2011 |
| | | JP | 5702723 B2 | 15-04-2015 |
| | | JP | 2012501724 A | 26-01-2012 |
| | | US | 2010055184 A1 | 04-03-2010 |
| | | US | 2016175483 A1 | 23-06-2016 |
| | | WO | 2010027471 A2 | 11-03-2010 |

EPO FORM P0459

For more details about this annex : see Official Journal of the European Patent Office, No. 12/82

## REFERENCES CITED IN THE DESCRIPTION

**Patent documents cited in the description**

- EP 1609491 B1
- EP 1181323 B1

- WO 2023145765 A1
- EP 3347061 B1


**Non-patent literature cited in the description**

- **TUGULU, S. et al.** RGD-functionalized polymer brushes as substrates for the integrin-specific adhesion of human umbilical vein endothelial cells. *Biomaterials*, 2007, vol. 28, 2536-2546
- **DAHLIN, C. et al.** Early Biocompatibility of Poly (Ethylene Glycol) Hydrogel Barrier Materials for Guided Bone Regeneration. *Clin. Oral Implants Res.*, 2014, vol. 25 (1), 16-20
- **FAN, L. et al.** The Use of Collagen-Based Materials in Bone Tissue Engineering. *Int. J. Mol. Sci.*, 2013, vol. 24, 3744
- **MEYER, M.** Processing of collagen based biomaterials and the resulting materials properties. *BioMed Eng OnLine.*, 2019, vol. 18 (24), 1-74

- IUPAC Compendium of Chemical Terminology. International Union of Pure and Applied Chemistry, 2006
- **EBRAHIMI, M.** Porosity parameters in biomaterial science: Definition, impact, and challenges in tissue engineering. *Front. Mater. Sci.*, 2021, vol. 15 (3), 352-373
- **LAURENT et al.** Fractionation of Hyaluronic Acid. The Polydispersity of Hyaluronic Acid from the Bovine Vitreous Body. *Biochim. Biophys. Acta*, 1960, vol. 42, 476-485
- **WECHSLER et al.** A novel, tissue occlusive poly(ethylene glycol) hydrogel material. *J. Biomed. Mater. Res.*, 2008, vol. 85A (2), 285-292